# EUROPEAN PATENT APPLICATION

(11) **EP 2 602 622 A1**
(43) Date of publication of application: **12.06.2013**
(21) Application number: 11192412.2
(22) Date of filing: 07.12.2011
(51) Int. Cl.: G01N 33/68

(54) **Prediction of response to platinum-based therapy**

(71) Applicant: Atlas Antibodies AB, 106 91 Stockholm (SE)
(72) Inventor: Jirström, Karin, 216 18 Limhamn (SE); Nodin, Björn, 247 56 Dalby (SE)
(74) Representative: Wirén, Anders

(57) **Abstract**

There is provided a method for determining whether a mammalian subject having a cancer belongs to a first or a second group, wherein subjects of the first group are more responsive to a platinum-based treatment than subjects of the second group, comprising the steps of:
a) evaluating an amount of Dachshund homolog 2 (DACH2) present in at least part of a sample earlier obtained from said subject, and determining a sample value corresponding to said amount;
b) comparing the sample value obtained in step a) with a reference value; and,
if said sample value is lower than or equal to said reference value,
c1) concluding that said subject belongs to said first group; and

if said sample value is higher than said reference value,
c2) concluding that said subject belongs to said second group. Corresponding means and uses are also provided.

## Description

### Technical field

The present invention relates to the field of cancer and in particular to treatment thereof. Further, it relates to means and methods useful in the establishment of a treatment prediction or an assessment of a cancer subject's responsiveness to a cancer treatment.

### Background

### Cancer

Cancer is one of the most common diseases, and a major cause of death in the western world. In general, incidence rates increase with age for most forms of cancer. As human populations continue to live longer, due to an increase of the general health status, cancer may affect an increasing number of individuals. The cause of most common cancer types is still largely unknown, although there is an increasing body of knowledge providing a link between environmental factors (dietary, tobacco smoke, UV radiation etc) as well as genetic factors (germ line mutations in "cancer genes" such as p53, APC, BRCA1, XP etc) and the risk for development of cancer.

No definition of cancer is entirely satisfactory from a cell biological point of view, despite the fact that cancer is essentially a cellular disease and defined as a transformed cell population with net cell growth and anti-social behavior. Malignant transformation represents the transition to a malignant phenotype based on irreversible genetic alterations. Although this has not been formally proven, malignant transformation is believed to take place in one cell, from which a subsequently developed tumor originates (the "clonality of cancer" dogma). Carcinogenesis is the process by which cancer is generated and is generally accepted to include multiple events that ultimately lead to growth of a malignant tumor. This multi-step process includes several rate-limiting steps, such as addition of mutations and possibly also epigenetic events, leading to formation of cancer following stages of precancerous proliferation. The stepwise changes involve accumulation of errors (mutations) in vital regulatory pathways that determine cell division, asocial behavior and cell death. Each of these changes may provide a selective Darwinian growth advantage compared to surrounding cells, resulting in a net growth of the tumor cell population. A malignant tumor does not only necessarily consist of the transformed tumor cells themselves but also surrounding normal cells, which act as a supportive stroma. This recruited cancer stroma consists of connective tissue, blood vessels and various other normal cells, e.g., inflammatory cells, which act in concert to supply the transformed tumor cells with signals necessary for continued tumor growth.

The most common forms of cancer arise in somatic cells and are predominantly of epithelial origin, e.g., prostate, breast, colon, urothelium and skin, followed by cancers originating from the hematopoetic lineage, e.g., leukemia and lymphoma, neuroectoderm, e.g., malignant gliomas, and soft tissue tumors, e.g., sarcomas.

### Cancer diagnostics and prognostics

Microscopic evaluation of biopsy material from suspected tumors remains the golden standard for cancer diagnostics. To obtain a firm diagnosis, the tumor tissue is fixated in formalin, histo-processed and paraffin embedded. From the resulting paraffin block, tissue sections can be produced and stained using both histochemical, i.e., hematoxylin-eosin staining, and immunohistochemical (IHC) methods. The surgical specimen is then evaluated with pathology techniques, including gross and microscopic analysis. This analysis often forms the basis for assigning a specific diagnosis, i.e., classifying the tumor type and grading the degree of malignancy, of a tumor.

Malignant tumors can be categorized into several stages according to classification schemes specific for each cancer type. The most common classification system for solid tumors is the tumor-node-metastasis (TNM) staging system. The T stage describes the local extent of the primary tumor, i.e., how far the tumor has invaded and imposed growth into surrounding tissues, whereas the N stage and M stage describe how the tumor has developed metastases, with the N stage describing spread of tumor to lymph nodes and the M stage describing growth of tumor in other distant organs. Early stages include: T0-1, N0, M0, representing localized tumors with negative lymph nodes. More advanced stages include: T2-4, N0, M0, localized tumors with more widespread growth and T1-4, N1-3, M0, tumors that have metastasized to lymph nodes and T1-4, N1-3, M1, tumors with a metastasis detected in a distant organ. Staging of tumors is often based on several forms of examination, including surgical, radiological and histopathological analyses. In addition to staging, for most tumor types there is also a classification system to grade the level of malignancy. The grading systems rely on morphological assessment of a tumor tissue sample and are based on the microscopic features found in a given tumor. These grading systems may be based on the degree of differentiation, proliferation and atypical appearance of the tumor cells. Examples of generally employed grading systems include Gleason grading for prostatic carcinomas and the Nottingham Histological Grade (NHG) grading for breast carcinomas.

Accurate staging and grading is crucial for a correct diagnosis and may provide an instrument to predict a prognosis. The diagnostic and prognostic information for a specific tumor subsequently determines an adequate therapeutic strategy for a given cancer patient. A commonly used method, in addition to histochemical staining of tissue sections, to obtain more information regarding a tumor is immunohistochemical staining. IHC allows for the detection of protein expression patterns in tissues and cells using specific antibodies. The use of IHC in clinical diagnostics allows for the detection of immunoreactivity in different cell populations, in addition to the information regarding tissue architecture and cellular morphology that is assessed from the histochemically stained tumor tissue section. IHC can be involved in supporting the accurate diagnosis, including staging and grading, of a primary tumor as well as in the diagnostics of metastases of unknown origin. The most commonly used antibodies in clinical practice today include antibodies against cell type "specific" proteins, e.g., PSA (prostate), MelanA (melanocytes) and Thyroglobulin (thyroid gland), and antibodies recognizing intermediate filaments (epithelial, mesenchymal, glial), cluster of differentiation (CD) antigens (hematopoetic, sub-classification of lympoid cells) and markers of malignant potential, e.g., Ki67 (proliferation), p53 (commonly mutated tumor suppressor gene) and HER-2 (growth factor receptor).

Aside from IHC, the use of *in situ* hybridization for detecting gene amplification and gene sequencing for mutation analysis are evolving technologies within cancer diagnostics. In addition, global analysis of transcripts, proteins or metabolites adds relevant information. However, most of these analyses still represent basic research and have yet to be evaluated and standardized for the use in clinical medicine.

### Platinum-based treatment

Platinum-based chemotherapy is used in treatment of cancers such as testicular, ovarian, cervical, lung, bladder, colorectal and head and neck cancer. Currently, there are three FDA-approved platinum-based compounds (cisplatin, carboplatin and oxaliplatin), but there are new derivatives being developed, or undergoing clinical trials (such as satraplatin and picoplatin). One goal in developing new platinum-based compounds is to minimize toxicity, which can be severe with the currently used compounds. Common side effects include kidney toxicity, nerve toxicity and loss of hearing.

### Platinum resistance

Platinum-based chemotherapeutic agents bind to DNA, thereby inducing DNA adducts, leading to cross-links that disrupt DNA structure. Usually, these damages ultimately lead to apoptosis. There is however a problem with drug resistance. A subset of tumors does not respond to conventional therapy, and this resistance could be either intrinsic or acquired.

The mechanism behind platinum resistance has not been fully elucidated, but two main pathways have been suggested: Either a failure of the platinum compound to reach the target DNA, or a failure of the platinum compound to induce apoptosis after the DNA adduct formation.

Failure of the platinum compound to reach tumor DNA could be due to the effect of certain proteins associated with multidrug resistance. There can also be other efflux proteins involved, for example those mediating copper transport.

A failure of the platinum compound to induce apoptosis is probably due to differences in DNA-repair systems. There are a number of mechanisms involved in DNA-repair, and particularly two of these have been associated with platinum resistance, Nucleotide excision repair (NER) and Mismatch repair (MMR).

With testicular cancer as a model system (these tumors are extremely sensitive to platinum-based chemotherapy), it has been shown that certain testicular carcinoma cell lines have a NER deficiency; in particular, low levels of excision repair cross-complementation group 1 (ERCC1) protein. In ovarian cancer cell lines it has been shown that cell lines developing cisplatin resistance had an increased ERCC1 expression.

There are indications that the MMR pathway needs to be functional in order for damages created by cisplatin and carboplatin to be detected by the cell. These compounds interfere with MMR activity, thus preventing damage repair, ultimately leading to apoptosis. If MMR is deficient, the cell can continue to proliferate with the DNA damage still present and will thereby be resistant. However, there seems to be a difference in MMR mediated resistance between different compounds; oxaliplatin may have effect in cells that are resistant to cisplatin and carboplatin.

### Endpoint analysis

Endpoint analysis for trials with adjuvant treatments for cancer gives important information on how the patients respond to a certain therapy. Overall survival (OS) has long been considered the standard primary endpoint. OS takes in to account time to death, irrespective of cause, e.g. if the death is due to cancer or not. Loss to follow-up is censored and regional recurrence, distant metastases, second primary cancers and second other primary cancers are ignored.

Today, an increasing number of effective treatments available for many types of cancer have resulted in the need for surrogate endpoints to allow for a better evaluation of the effect of adjuvant treatments. Partly due to the long follow-up period required to demonstrate that adjuvant treatments improve OS, the endpoint is often complemented with other clinical endpoints that give an earlier indication on how successful the treatment is.

In the present disclosure, one surrogate endpoint is used, namely ovarian cancer-specific survival (OCSS). Analysis of OCSS includes time to death caused by ovarian cancer due to the original tumor. RFS, which also may be used, includes time to any event related to the same cancer, i.e., all cancer recurrences and deaths from the same cancer are events. Distant, local and regional metastases as well as ovarian cancer specific death are considered. On the other hand, second primary same cancers and other primary cancers are ignored. Deaths from other cancers, non-cancer-related deaths, treatment-related deaths, and loss to follow-up are censored observations.

### Summary

There is an object of some aspects of the present disclosure to provide for the prediction of the level of responsiveness to platinum-based treatment.

The following is a non-limiting and itemized listing of embodiments of the present disclosure, presented for the purpose of providing various features and combinations provided by the invention in certain of its aspects.
1. Method for determining whether a mammalian subject having a cancer belongs to a first or a second group, wherein subjects of the first group are more responsive to a platinum-based treatment than subjects of the second group, comprising the steps of:
   a) evaluating an amount of Dachshund homolog 2 (DACH2) present in at least part of a sample earlier obtained from said subject, and determining a sample value corresponding to said amount;
   b) comparing the sample value obtained in step a) with a reference value; and,
      if said sample value is lower than or equal to said reference value,
      c1) concluding that said subject belongs to said first group; and if said sample value is higher than said reference value,
      c2) concluding that said subject belongs to said second group.
2. Method for determining the level of intensity of a platinum-based treatment of a mammalian subject having a cancer, comprising the steps of:
   a) evaluating an amount of DACH2 present in at least part of a sample earlier obtained from said subject, and determining a sample value corresponding to said amount;
   b) comparing the sample value obtained in step a) with a reference value; and,
      if said sample value is lower than or equal to said reference value, c1) concluding that said subject should be given a platinum-based treatment of said first intensity; and
      if said sample value is higher than or equal to said reference value, c2) concluding that said subject should be given a platinum-based treatment of said second intensity,
      wherein said second intensity is higher than said first intensity.
3. Method of treatment of a mammalian subject having a cancer, comprising the steps of:
   a) evaluating an amount of DACH2 present in at least part of a sample from said subject, and determining a sample value corresponding to said amount;
   b) comparing the sample value obtained in step a) with a reference value; and,
      if said sample value is lower than or equal to said reference value,
   c) applying a platinum-based treatment to said subject.
4. Method according to any one of items 1-3, wherein said cancer is selected from testicular, ovarian, lung, bladder, colorectal, cervical, breast and head and neck cancer.
5. Method according to item 4, wherein said cancer is selected from testicular, ovarian, bladder, colorectal, cervical and breast cancer.
6. Method according to any one of the preceding items, wherein said platinum-based treatment is selected from carboplatin, paraplatin, oxaliplatin, satraplatin, picoplatin and cisplatin treatment.
7. Method according to any one of the preceding items, wherein said sample is a body fluid sample.
8. Method according to item 7, wherein the body fluid is selected from the group consisting of blood, plasma, serum, seminal fluid, semen, cerebral fluid, urine and exudate.
9. Method according to any one of items 1-6, wherein said sample is a cytology sample.
10. Method according to any one of items 1-6, wherein said sample is a stool sample.
11. Method according to any one of the preceding items, wherein said sample comprises cells from said subject.
12. Method according to any one of the preceding items, wherein said sample comprises tumor cells from said subject.
13. Method according to any one of items 1-6, wherein said sample is a tissue sample.
14. Method according to item 13, wherein said tissue sample is a tumor tissue sample.
15. Method according to any one of items 11-14, wherein the evaluation of step a) is limited to the nuclei of cells of said sample.
16. Method according to item 15, wherein the evaluation of step a) is limited to the nuclei of tumor cells of said sample.
17. Method according to any preceding item, wherein the sample value of step a) is determined as being either 1, corresponding to detectable DACH2 in the sample, or 0, corresponding to no detectable DACH2 in the sample.
18. Method according to any preceding item, wherein the reference value of step b) corresponds to a reference sample having no detectable DACH2.
19. Method according to any preceding item, wherein the reference value of step b) is 0.
20. Method according to any one of the preceding items, wherein said reference value is a nuclear fraction, a nuclear intensity or a function of a nuclear fraction and a nuclear intensity.
21. Method according to any one of the preceding items, wherein DACH2 is a protein and its amino acid sequence comprises a sequence selected from:
   i) SEQ ID NO:1; and
   ii) a sequence which is at least 85 % identical to SEQ ID NO:1.
22. Method according to any one of the preceding items, wherein DACH2 is a protein and its amino acid sequence comprises or consists of a sequence selected from:
   i) SEQ ID NO:2, 3, 4 or 5; and
   ii) a sequence which is at least 85 % identical to SEQ ID NO:2, 3, 4 or 5.
23. Method according to any one of the preceding items, wherein step a) comprises:
   aI) applying to said sample a quantifiable affinity ligand capable of selective interaction with a DACH2 protein to be evaluated, said application being performed under conditions that enable binding of the affinity ligand to DACH2 protein present in the sample; and
   aII) quantifying the affinity ligand bound to said sample to evaluate said amount.
24. Method according to any one of items 1-22, wherein step a) comprises:
   aI) applying to said sample a quantifiable affinity ligand capable of selective interaction with a DACH2 protein to be quantified, said application being performed under conditions that enable binding of the affinity ligand to DACH2 protein present in the sample;
   a2) removing non-bound affinity ligand; and
   a3) quantifying affinity ligand remaining in association with the sample to evaluate said amount.
25. Method according to item 23 or 24, wherein the quantifiable affinity ligand is selected from the group consisting of antibodies, fragments thereof and derivatives thereof.
26. Method according to item 25, wherein said quantifiable affinity ligand is obtainable by a process comprising a step of immunizing an animal with a peptide whose amino acid sequence consists of SEQ ID NO:1.
27. Method according to item 23 or 24, wherein said quantifiable affinity ligand is an oligonucleotide molecule.
28. Method according to item 23 or 24, wherein the quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.
29. Method according to any one of items 23-28, wherein said quantifiable affinity ligand is capable of selective interaction with a peptide whose amino acid sequence consists of a sequence SEQ ID NO:1.
30. Method according to any one of items 1-19, wherein step a) comprises evaluation of the amount of DACH2 mRNA by means of a microarray, a PCR amplification (such as RT quantitative PCR), a RNA hybridization, a gel electrophoresis or a combination thereof to obtain said amount.
31. Kit comprising:
   a) a quantifiable affinity ligand capable of selective interaction with a DACH2 protein; and
   b) reagents necessary for quantifying the amount of said quantifiable affinity ligand.
32. Kit according to item 31, in which said quantifiable affinity ligand is selected from the group consisting of antibodies, fragments thereof and derivatives thereof.
33. Kit according to item 32, in which said quantifiable affinity ligand is obtainable by a process comprising a step of immunizing an animal with a peptide whose amino acid sequence consists SEQ ID NO:1.
34. Kit according to item 31, in which said quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.
35. Kit according to item 31, in which said quantifiable affinity ligand is an oligonucleotide molecule.
36. Kit according to any one of items 31-35, in which said quantifiable affinity ligand is capable of selective interaction with a DACH2 fragment consisting of SEQ ID NO:1.
37. Kit according to any one of items 31-36, in which said reagents necessary for quantifying said amount of said quantifiable affinity ligand comprise a secondary affinity ligand capable of recognizing said quantifiable affinity ligand.
38. Kit according to item 37, in which said secondary affinity ligand comprises a label selected from the group consisting of fluorescent dyes or metals, chromophoric dyes, chemiluminescent compounds and bioluminescent proteins, enzymes, radioisotopes, particles and quantum dots.
39. Kit according to any one of items 31-38, comprising:
   a first reference sample comprising an amount of DACH2 protein corresponding to a value (positive reference value) being higher than a reference value; and
   a second reference sample comprising an amount of DACH2 protein corresponding to a value (negative reference value) being lower than or equal to said reference value.
40. Kit according to any one of items 31-39, in which said reference samples comprises a cell line.
41. Use *ex vivo* of a DACH2 protein as a treatment predictive marker for platinum-based treatment of a mammalian subject having a cancer.
42. Use *ex vivo* of a DACH2 protein or an antigenically active fragment thereof for the selection or purification of a treatment predictive agent for platinum-based treatment of a mammalian subject having a cancer.
43. Use of a DACH2 protein or an antigenically active fragment thereof for the production of a treatment predictive agent for platinum-based treatment of a mammalian subject having a cancer.
44. Use according to item 42 or 43, wherein said treatment predictive agent is an affinity ligand capable of selective interaction with the DACH2 protein or an antigenically active fragment thereof.
45. Use according any one of items 41-44, wherein the amino acid sequence of the DACH2 protein or antigenically active fragment thereof comprises a sequence selected from:
   i) SEQ ID NO:1; and
   ii) a sequence which is at least 85 % identical to SEQ ID NO:1.
46. Use according any one of items 41-44, wherein the amino acid sequence of the DACH2 protein comprises or consists of a sequence selected from:
   i) SEQ ID NO:2, 3, 4 or 5; and
   ii) a sequence which is at least 85 % identical to SEQ ID NO:2, 3, 4 or 5.
47. Affinity ligand, which is obtainable by a process comprising a step of immunizing an animal with a peptide whose amino acid sequence consists of sequence SEQ ID NO:1.
48. Affinity ligand capable of selective interaction with a peptide whose amino acid sequence consists of SEQ ID NO:1.
49. Affinity ligand according to item 47 or 48, which is a monoclonal or polyclonal antibody.
50. Use *ex vivo* of an affinity ligand capable of selective interaction with a DACH2 protein as a treatment predictive agent for platinum-based treatment of a mammalian subject having a cancer.
51. Use *ex vivo* of an affinity ligand capable of selective interaction with a DACH2 protein for indicating whether a mammalian subject having a cancer should be given a platinum-based treatment.
52. Use of an affinity ligand capable of selective interaction with a DACH2 protein in the manufacture of a treatment predictive agent for platinum-based treatment of a mammalian subject having a cancer.
53. Use according to any one of items 50-52, wherein the affinity ligand is an affinity ligand according to any one of items 47-49.
54. Platinum-based therapeutic agent for use in treatment of a mammalian subject having a cancer, wherein said subject is diagnosed as DACH2 low.
55. Platinum-based therapeutic agent according to item 54 being cisplatin.
56. Platinum-based therapeutic agent according to item 54-55, wherein the cancer is selected from testicular, ovarian, lung, bladder, colorectal, cervical, breast and head and neck cancer.
57. Use of a platinum-based therapeutic agent in the manufacture of a medicament for treatment of a mammalian subject having a cancer, wherein said subject is diagnosed as DACH2 low.

### Brief description of the figures

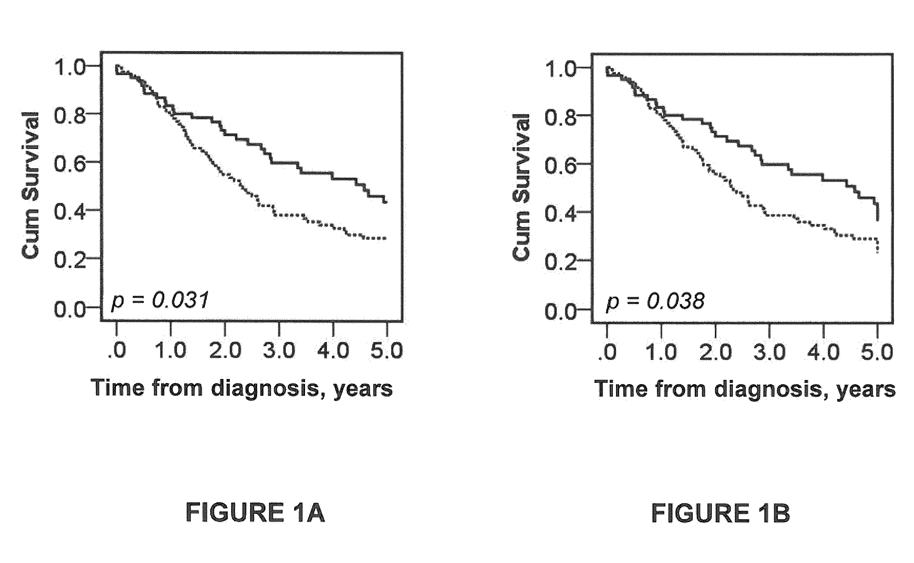

Figure 1 shows the impact of DACH2 level on survival over five years in ovarian cancer subjects who are very likely to have been treated by platinum-based chemotherapy. A nuclear score of higher than 3 is considered "high level" and is represented by the dotted line, while a nuclear score of 3 or lower is considered "low level" and is represented by a solid line. Figure 1A shows overall survival (OS) and Figure 1B shows ovarian cancer specific survival (OCSS).

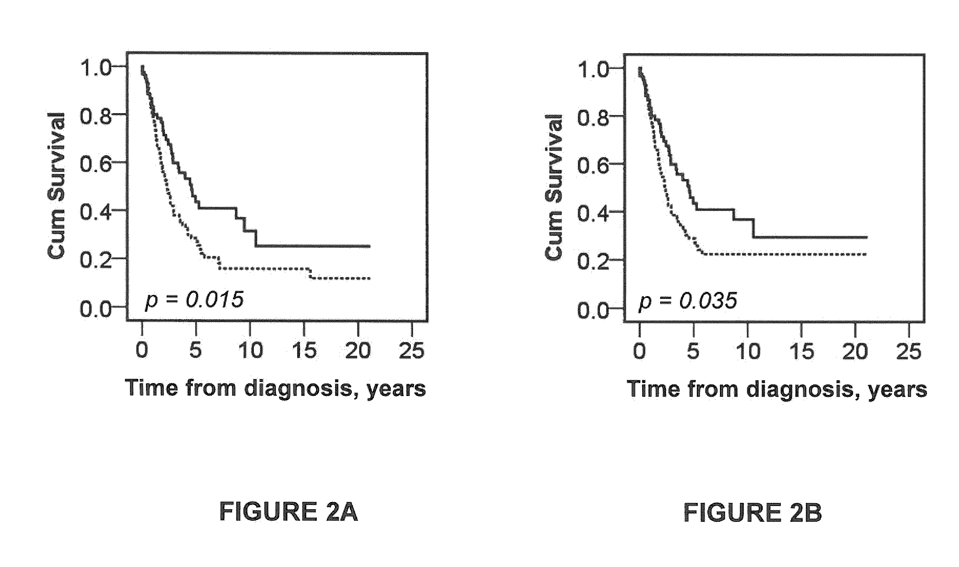

Figure 2 shows the impact of DACH2 level on survival (follow up time: over 20 years) in ovarian cancer subjects who are very likely to have been treated by platinum-based chemotherapy. A nuclear score of higher than 3 is considered "high level" and is represented by the dotted line, while a nuclear score of 3 or lower is considered "low level" and is represented by a solid line. Figure 2A shows overall survival (OS) and Figure 2B shows ovarian cancer specific survival (OCSS).

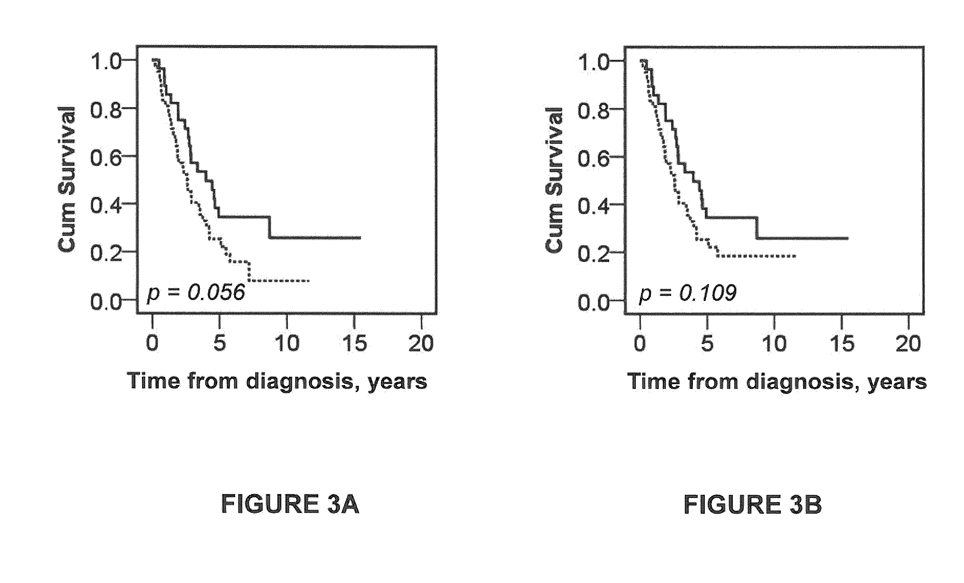

Figure 3 shows the impact of DACH2 level on survival in ovarian cancer subjects who are confirmed to have been treated by platinum-based chemotherapy. A nuclear score of higher than 3 is considered "high level" and is represented by the dotted line, while a nuclear score of 3 or lower is considered "low level" and is represented by a solid line. Figure 3A shows overall survival (OS) and Figure 3B shows ovarian cancer specific survival (OCSS).

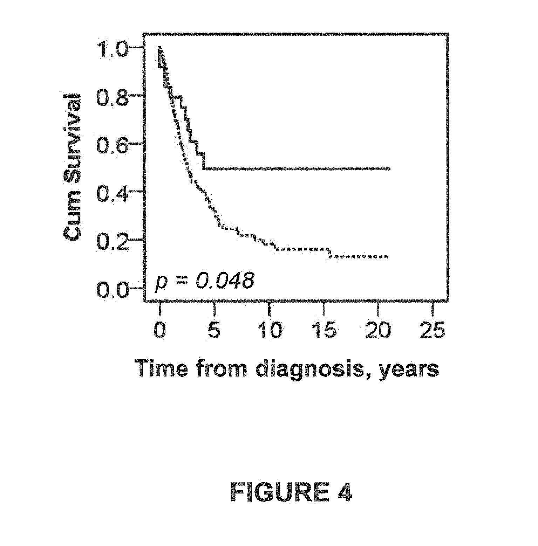

Figure 4 shows the impact of DACH2 level on overall survival (OS) in ovarian cancer subjects who are very likely to have been treated by platinum-based chemotherapy. A nuclear fraction of 2 or 3 (> 25 %) is considered "high level" and is represented by the dotted line, while a nuclear fraction of 0 or 1 (≤ 25 %) is considered "low level" and is represented by a solid line.

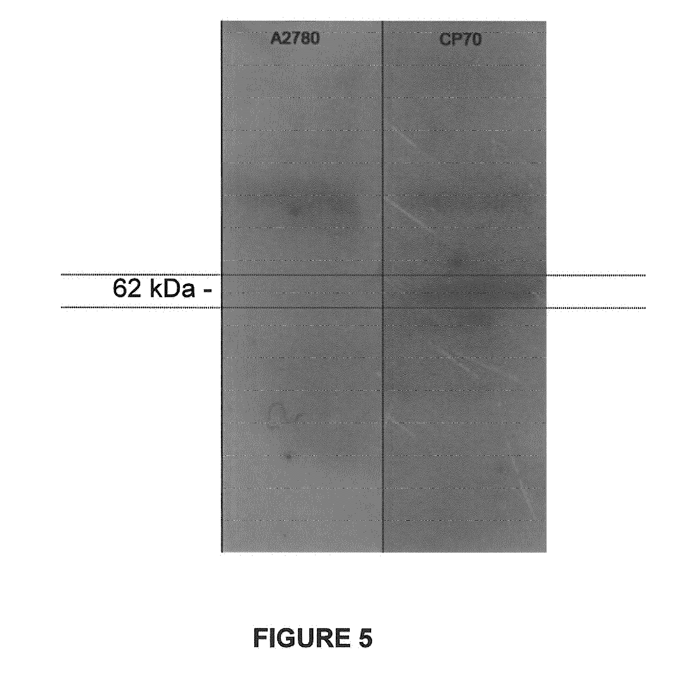

Figure 5 shows a Western Blot analysis of DACH2 expression in lysed cisplatin sensitive (A2780) and cisplatin resistant (CP70) cells. 62 kDa is the molecular weight of a DACH2 protein.

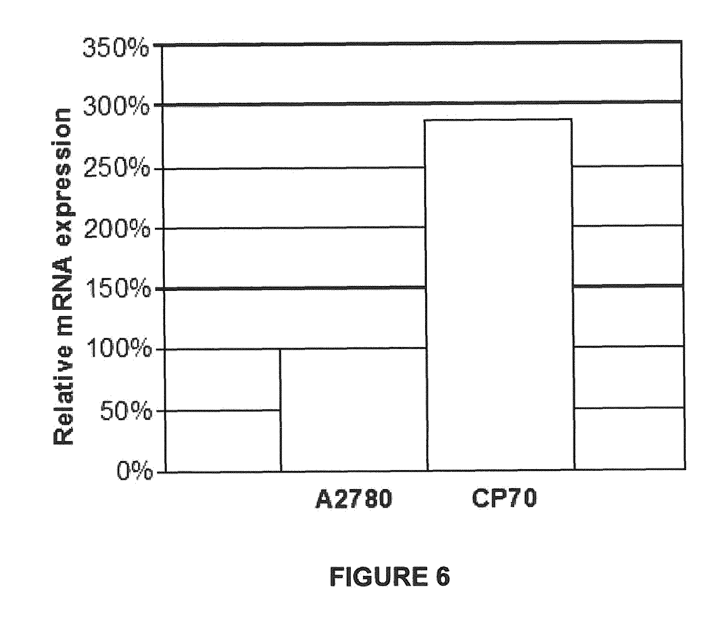

Figure 6 shows normalized values of DACH2 mRNA expression in cisplatin sensitive (A2780) and cisplatin resistant (CP70) cells.

### Detailed description

As a first aspect of the present disclosure, there is provided a method for determining whether a mammalian subject having a cancer belongs to a first or a second group, wherein subjects of the first group are more responsive to a platinum-based treatment than subjects of the second group, comprising the steps of:
a) evaluating an amount of Dachshund homolog 2 (DACH2) present in at least part of a sample earlier obtained from said subject, and determining a sample value corresponding to said amount;
b) comparing the sample value obtained in step a) with a reference value; and,
if said sample value is lower than or equal to said reference value,
c1) concluding that said subject belongs to said first group; and if said sample value is higher than said reference value,
c2) concluding that said subject belongs to said second group.

The first group differs from the second group in that the subjects of the first group generally are more responsive to platinum-based treatment than the subjects of the second group. That means that subjects of the first group generally have a higher probability of survival if given a platinum-based treatment regimen than subjects of the second group if given the same treatment regimen or that subjects of the first group generally require lower doses to reach a given probability of survival than subjects of the second group. Here, survival may be measured as overall survival, recurrence-free survival or cancer type specific survival.

It is thus determined whether a cancer subject belongs to a first or a second group, wherein subjects of the first group generally are more responsive to platinum-based treatment than subjects of the second group. The division of subjects having a given cancer into the two groups is determined by comparing samples values from the subjects with a reference value. The reference value is thus the determinant for the size of the respective groups; the lower the reference value, the fewer the subjects in the first group and the lower the likelihood that a tested subject belongs to the first group.

For the avoidance of doubt, the first and the second group consist of subjects having the same type of cancer as the tested subject. Ovarian cancer subjects are thus not compared to or grouped with subjects having testicular cancer. Further, the first and the second group may consist of subjects having the same or similar stage and/or subtype of cancer as the tested subject. Also, the groups may consist of subjects having the same or similar age, race, sex, menopausal status, genetic characteristics and/or medical status or history as the tested subject.

The present disclosure is based on the inventors' insight that the expression of DACH2 in a sample obtained from a subject having a cancer may serve as an indicator of the subject's response to platinum-based treatment. The inventors have identified a correlation between values of DACH2 on the one hand and response to platinum-based therapeutic treatment on the other. Typically, low DACH2 values are shown herein to correlate with a relatively high responsiveness to platinum-based treatment.

Platinum-based treatment has proven effective in a range of cancer types. However, the treatment is normally associated with, sometimes severe, side-effects. Therefore, the inventors have concluded that it is beneficial to adapt the doses of the treatment (i.e. the intensity of the treatment) to the responsiveness of the cancer subject such that unnecessary over-treatment is avoided. That is, a highly responsive subject may need a lower dose than a less responsive subject. Further, it is beneficial to identify the subjects who are unlikely to at all respond to the treatment. Such subjects should be treated with alternative regimens.

In conclusion, the above method may assist the physician responsible for the treatment of a cancer subject when deciding whether to apply platinum-based treatment or not and, in cases when platinum-based treatment is found appropriate, the intensity level of the treatment.

If the cancer subject of the above method is found to belong to the first group, the physician's decision may thus be to apply a platinum-based treatment at a relatively low dose. If, however, the cancer subject is found to belong to the second group, the physicians' decision may be to apply the platinum-based treatment at a relatively high dose or to refrain from the platinum-based treatment, possibly in favor of another treatment.

Accordingly, as a second aspect of the present disclosure, there is provided a method for determining the level of intensity of a platinum-based treatment of a mammalian subject having a cancer, comprising the steps of:
a) evaluating an amount of DACH2 present in at least part of a sample earlier obtained from said subject, and determining a sample value corresponding to said amount;
b) comparing the sample value obtained in step a) with a reference value; and,
if said sample value is lower than or equal to said reference value, c1) concluding that said subject should be given a platinum-based treatment of said first intensity; and
if said sample value is higher than or equal to said reference value, c2) concluding that said subject should be given a platinum-based treatment of said second intensity,
wherein said second intensity is higher than said first intensity.

The above method of the second aspect may be particularly relevant for subjects having ovarian cancer or testicular cancer, which are almost always treated with platinum-based therapy in many parts of the world today.

The level of intensity may for example be measured as the average daily or weekly dose of a platinum-based therapeutic agent given to the subject. A treatment of the second intensity may thus be applied more frequently or in higher individual doses than a treatment of the first intensity. The treatment of the second intensity may also comprise application of a more aggressive platinum-based therapeutic agent than the treatment of the first intensity. Yet another possibility is that the treatment of the second intensity is applied for a longer period than the treatment of the first intensity.

In an embodiment of the second aspect, c1) may thus be concluding that said subject should undergo platinum-based treatment during a first period and c2) may be concluding that said subject should undergo platinum-based treatment during a second period, wherein the second period is longer than the first period.

As an alternative configuration of the second aspect, there is provided a method for determining whether to refrain from a platinum-based treatment of a mammalian subject having a cancer, comprising the steps of:
a) evaluating an amount DACH2 present in at least part of a sample earlier obtained from said subject, and determining a sample value corresponding to said amount;
b) comparing the sample value obtained in step a) with a reference value; and,
   if said sample value is higher than said reference value,
c) refraining from treating the subject with the platinum-based treatment.

This alternative embodiment of the second aspect is particularly relevant for subjects having cancers for which platinum-based treatment is not considered standard, e.g. all cancers but ovarian cancer and testicular cancer or cancers selected from the group consisting of lung, bladder, colorectal, cervical, breast and head and neck cancer.

In any case, DACH2 low cancer subjects are likely to benefit from platinum-based treatment according to teachings of the present disclosure. As a third aspect of the present disclosure, there is thus provided a method of treatment of a mammalian subject having a cancer, comprising the steps of:
a) evaluating an amount of DACH2 present in at least part of a sample from said subject, and determining a sample value corresponding to said amount;
b) comparing the sample value obtained in step a) with a reference value; and,
   if said sample value is lower than or equal to said reference value,
c) applying a platinum-based treatment to said subject.

The method of treatment may be limited to the decision-making and treatment. Thus, as a configuration of the third aspect, there is provided a method of treatment of a subject having a cancer, comprising:
α) comparing a sample value corresponding to a level of DACH2 in a sample from the subject with a reference value; and,
if said sample value is lower than or equal to said reference value,
β) applying a platinum-based treatment to said subject.

Numerous ways of obtaining a sample value corresponding to a level of DACH2 in a sample from a subject are described in the present disclosure, which also provides different examples of (clinically) relevant reference values.

Regarding step a) of the methods of the present disclosure, an increase in the amount of DACH2 typically results in an increase in the sample value, and not the other way around. However, in some embodiments, the evaluated amount may correspond to any of a predetermined number of discrete sample values. In such embodiments, a first amount and a second, increased, amount may correspond to the same sample value. In any case, an increase in the amount of DACH2 will not result in a decrease in the sample value in the context of the present disclosure.

However inconvenient, but in an equivalent fashion, the evaluated amounts may be inversely related to sample values if the qualification between step b) and c) is inverted. For example, the qualification between step b) and c) is inverted if the phrase "if the sample value is lower than or equal to the reference value" is replaced with "if the sample value is higher than or equal to the reference value".

A physician responsible for the treatment of a cancer subject may take several parameters into account, such as the result of an immunohistochemical evaluation, patient age, hormone receptor status, general condition of the patient, medical history, such as cancer history and hereditary characteristics, e.g. whether there is a history of cancer in the subject's family when deciding on a suitable treatment regimen for the subject. To be guided in the decision, the physician may perform a DACH2 test, or order a DACH2 test performed, according to anyone of the above methods.

The steps of the above methods may be performed by a single person, such as a lab technician, nurse or physician. However, a physician may also perform step c) and optionally step b) himself, while assigning the performance of step a) and optionally step b) to someone else, typically a lab technician or a nurse. Also, the physician responsible for the diagnosis and treatment may be a physician specializing in the cancer in question, while the physician performing the examination of the sample has another area of expertise, such as pathology. If the above methods involve more than one person, the persons may be situated at different locations and have different employers. For example, the physician may be employed by a hospital while the lab technician or pathologist is employed by a lab. Samples and information may thus be transferred from one person and location to another person and location within the framework of the methods of the present disclosure.

As a variant of the above methods, there is provided a method for predicting the response of a platinum-based treatment of a mammalian subject having a cancer, comprising the steps of:
a) evaluating an amount of DACH2 present in at least part of a sample from the subject, and determining a sample value corresponding to the evaluated amount;
b) correlating the sample value of step a) to a predicted response to the treatment.

In one embodiment, the sample may be an earlier obtained sample.

The correlating of step b) refers to any way of associating response data, such as survival data, to the obtained sample value so as to establish the treatment prediction. With the knowledge of the teachings of the present disclosure, the skilled person may conclude a predicted response without undue burden.

In the context of the methods of the present disclosure, "earlier obtained" refers to obtained before the method is performed. Consequently, if a sample earlier obtained from a subject used in a method, the method does not involve obtaining the sample from the subject, i.e., the sample was previously obtained from the subject in a step separate from the method.

All the methods and uses of the present disclosure, except the methods of treatment, may be carried out entirely *ex vivo* unless otherwise stated or indicated.

Further, in the context of the present disclosure, "a mammalian subject having a cancer" refers to a mammalian subject having a primary or secondary tumor or a mammalian subject which has had such tumor eradicated/removed, wherein the eradicating/removal of the tumor refers to killing or eradicating/removing the tumor by any type of surgery or therapy. In the latter case, the tumor may for example have been eradicated/removed less than one year ago. For example, a subject who has had a tumor eradicated by surgery and is about to get adjuvant therapy is considered "having a cancer" in the context of the present disclosure. In the method and use aspects of the present disclosure, "a mammalian subject having a cancer" also includes the case wherein the mammalian subject is suspected of having a cancer at the time of the performance of the use or method and the cancer diagnosis is established later.

Still further, in the context of the present disclosure, the "reference value" refers to a predetermined value which is relevant for making decisions, or drawing conclusions, regarding the treatment or treatment prediction. Guided by the teachings of the present disclosure, the person skilled in the art may select a relevant reference value without undue burden.

Step a) of the methods of the above aspects involve evaluating an amount of DACH2 present in at least part of the sample, and determining a sample value corresponding to the amount. The "at least part of the sample" refers to a relevant part or relevant parts of the sample for establishing a level of responsiveness or drawing conclusions regarding suitable treatments. The person skilled in the art understands which part or parts that are relevant under the circumstances present when performing the method. For example, if evaluating a sample comprising cells, the skilled person may only consider the tumor cells, or only the nuclei of tumor cells, of the sample.

Further, in step a) an amount is evaluated and a sample value corresponding to the amount is determined. Consequently, an exact measurement of the amount of DACH2 is not required for obtaining the sample value. For example, the amount of DACH2 protein may be evaluated by visual inspection of a prepared and stained tissue sample and the sample value may then be categorized as for example high or low based on the evaluated amount.

The person of skill in the art understands that the evaluation and determination of step a) involves some kind of processing or manipulation of the sample obtained from the subject. It is not possible to determine the sample value by mere visual inspection of the native sample. Various techniques, of which some are presented below, for such evaluation and determination are well known to the skilled person. The methods of the present disclosure are therefore not limited to any specific technique or techniques for the performance of step a).

In Examples below, the level of DACH2 expression is shown to correlate with survival in ovarian cancer subjects treated with a platinum-based therapeutic agent. The finding of the differential expression of the DACH2 and the treatment predictive relevance thereof thus forms the basis of the present disclosure. In addition to ovarian cancer, the DACH2 protein is differentially expressed in a number of cancer types, as shown in table 1 (Examples, section 1). Accordingly, the inventors conclude that the finding in ovarian cancer is likely to extend to other cancer types. In particular, it is notable that the DACH2 protein is shown to be differentially expressed in testicular, ovarian, bladder, colorectal, cervical and breast cancer, which are all cancer types considered suitable for platinum-based treatment today.

In embodiments of the present disclosure, the cancer is selected from the group consisting of testicular, ovarian, lung, bladder, colorectal, cervical and head and neck cancer (urothelial cancer is a type of bladder cancer). Platinum-based treatment has been found suitable for subjects having such cancers in clinical trials.

In preferred embodiments of the present disclosure, the cancer is selected from the group consisting of testicular, ovarian, bladder, colorectal, cervical and breast cancer.

In some embodiments, the cancer is selected from ovarian cancer and testicular cancer. Subjects having ovarian cancer or testicular cancer are almost always given platinum-based therapy in many parts of the world, and the findings of the present disclosure are thus particularly relevant in these cancer types.

If the cancer of the present disclosure is an ovarian cancer, it may according to one embodiment be an epithelial ovarian cancer, such as a serous ovarian cancer. The inventors have noted that, in some experiments, the association between DACH2 expression and outcome of platinum-based treatment has been particularly accentuated for subjects having serous ovarian cancers.

If the cancer of the present disclosure is a colorectal cancer, it may according to one embodiment be a colorectal cancer of a relatively advanced stage, such as Dukes' stage C or D. However, Dukes' stage B cancers may also be relevant since there is a risk of recurrence in cancer of that stage. Thus, DACH2 low Dukes' stage B subjects may be given platinum-based treatment to prevent recurrence. The colorectal cancer may also be of stage II, i.e. stage IIA (T3, N0, M0), stage IIB (T4a, N0, M0) or stage IIC (T4b, N0, M0).

If the cancer of the present disclosure is a breast cancer, it may according to one embodiment be a triple negative breast cancer, i.e. a breast cancer negative for the estrogen receptor, progesterone receptor and HER2 receptor. Triple negative breast cancer subjects are considered particularly difficult to treat today and there are ongoing clinical trials in which such subjects are treated with the platinum-based agent cisplatin.

A platinum-based treatment comprises application of platinum-based therapeutic agent. Carboplatin (Paraplatin), oxaliplatin, satraplatin, picoplatin and cisplatin are some platinum-based therapeutic agents tested or used in the clinic today. Cisplatin, which is employed in Examples below, is widely used in the clinic, e.g. for the treatment of ovarian cancer, small cell lung cancer and testicular cancer. Oxaliplatin is used in the clinic for treatment of colorectal cancer, often in combination with fluorouracil and leucovorin. Carboplatin is also used in the clinic, e.g. for treatment of ovarian, lung and head and neck cancer. In lung cancer, it is often used in combination with gemcitabine.

In embodiments of the present disclosure, the platinum-based treatment may thus be application of an agent selected from carboplatin, oxaliplatin, satraplatin, picoplatin and cisplatin. In embodiments wherein the cancer is ovarian or lung cancer, the agent may for example be cisplatin or carboplatin. In embodiments where the cancer is colorectal cancer, the agent may for example be oxaliplatin. In embodiments where the cancer is testicular cancer, the agent may for example be cisplatin.

In embodiments of the methods of the above aspects, the sample may be a body fluid sample. For example, the body fluid sample may be selected from the group consisting of blood, plasma, serum, cerebral fluid, urine, lymph, seminal fluid and exudate. Alternatively, the sample may be a cytology sample or a stool sample.

The level of DACH2 protein may preferably be measured intracellularly or in cell-derived material. Thus, the body fluid, cytology or stool sample may for example comprise cells, such as tumor cells.

In further embodiments of the methods of the above aspects, the sample may be a tissue sample, such as a tumor sample, e.g, from a previously performed biopsy or surgical removal of a tumor from the subject.

Further, the inventors have noted that nuclear expression of DACH2 protein is relevant for determining the level of responsiveness to platinum-based treatment or selecting treatments. Thus, the evaluation of step a) may be limited to the nuclei of cells, such as tumor cells, of said sample. Consequently, when a tissue sample is examined, only the nuclei of tumor cells may be taken into consideration. Such examination may for example be aided by immunohistochemical staining.

When performing the methods according to the above aspects, it may be convenient to use zero as the reference value, because in such case, it has only to be established in step a) whether DACH is present in the relevant parts of the sample or not.

Thus, in embodiments of the methods of the above aspects, the sample value of step a) may be either 1, corresponding to detectable DACH2 in the sample, or 0, corresponding to no detectable DACH2 in the sample. Consequently, in such embodiments, the evaluation of the sample is digital: DACH2 is considered to be either present or not. In the context of the present disclosure, "no detectable DACH2" refers to an amount of DACH2 that is so small that it is not, during normal operational circumstances, detectable by a person or an apparatus performing the step a). The "normal operational circumstances" refer to the laboratory methods and techniques a person skilled in the art would find appropriate for performing the methods of the present disclosure.

Accordingly, in embodiments of the methods of the present disclosure, the reference value of step b) may be 0. And it follows that, in further embodiments of the methods of the present disclosure, the reference value of step b) may correspond to a reference sample having no detectable DACH2 (see below).

A DACH2 sample value being higher than the reference value, or a subject from which such sample value is obtained, is sometimes referred to herein as being "DACH2 high". Further, a DACH2 sample value being lower than or equal to the reference value, or a subject from which such sample value is obtained, is sometimes referred to herein as being "DACH2 low".

In the context of the present disclosure, the terms "sample value" and "reference value" are to be interpreted broadly. The quantification of DACH2 to obtain these values may be done via automatic means, via a scoring system based on visual or microscopic inspection of samples or via combinations thereof. However, it is also possible for a skilled person, such as a person skilled in the art of histopathology, to determine the sample and reference values by inspection, e.g., of tissue slides that have been prepared and stained for DACH2 protein expression.

Determining that the sample value is higher than the reference value may thus be determining, upon visual or microscopic inspection, that a sample tissue slide is more densely stained and/or exhibit a larger fraction of stained cells than a reference tissue slide. The sample value may also be compared to a reference value given by a literal reference, such as a reference value described in wording or by a reference picture. Consequently, the sample and/or reference values may in some cases be mental values that the skilled person envisages upon inspection and comparison.

One or more of the steps of the methods of the present disclosure may be implemented in an apparatus. For example, step a) and optionally step b) may be performed in an automatic analysis apparatus, and such an apparatus may be based on a platform adapted for immunohistochemical analysis. As an example, one or more tumor tissue sample(s) from the subject in question may be prepared for imunohistochemical analysis manually and then loaded into the automatic analysis apparatus, which gives the sample value of step a) and optionally also performs the comparison with the reference value of step b). The operator performing the analysis, the physician ordering the analysis or the apparatus itself may then draw the conclusion of step c). Consequently, software adapted for drawing the conclusion of step c) may be implemented on the apparatus.

Further, step a) may for example comprise staining of the sample in an automatic staining apparatus, such as BenchMark XT or BenchMark Ultra marketed by Ventana

A reference value, found to be relevant for establishing treatment prediction or making treatment decisions regarding cancer subjects, for use as comparison with the sample value from the subject, may be provided in various ways. With the knowledge of the teachings of the present disclosure, the skilled artisan can, without undue burden, provide relevant reference values for performing the methods of the present disclosure.

The person performing the methods of the above aspects may, for example, adapt the reference value to desired information. For example, the reference value may be adapted to yield the most significant information with regard to survival of subjects given platinum-based treatment, e.g., the largest separation between the DACH2 low survival curve and the DACH2 high survival curve (see the figures), which corresponds to the largest difference in survival between the first and the second group of the first aspect. Alternatively, the reference value may be selected such that a group of subjects having particularly high responsiveness or particularly low responsiveness is singled out.

In embodiments of the methods of the above aspects, the reference value may correspond to the amount of DACH2 measured in a reference sample comprising tumor cells, such as a reference sample of tumor tissue. The amount of protein expression of the reference sample may preferably be previously established. Consequently, the reference value may be provided by the amount of DACH2 measured in a reference sample comprising cells expressing a predetermined amount of DACH2.

Further, the reference value may for example be provided by the amount of DACH2 measured in a reference sample comprising cell lines, such as cancer cell lines, expressing a predetermined, or controlled, amount of DACH2. The person skilled in the art understands how to provide such cell lines, for example guided by the disclosure of Rhodes et al. (2006) The biomedical scientist, p 515-520. Cell lines expressing different amounts of DACH2 are also described in the Examples below.

However, as discussed further below, the amount of DACH2 protein in the reference sample does not have to directly correspond to the reference value. The reference sample may also provide an amount of DACH2 protein that helps a person performing the method to assess various reference values. For example, the reference sample(s) may help in creating a mental image of the reference value by providing a "positive" reference value and/or a "negative" reference value.

One alternative for the quantification of DACH2 protein expression in a sample, such as the sample earlier obtained from the subject or the reference sample, is the determination of the fraction of cells in the sample that exhibit DACH2 protein expression over a certain level. The fraction may for example be: a "cellular fraction", wherein the DACH2 protein expression of the whole cells is taken into account; a "cytoplasmic fraction", wherein the DACH2 protein expression of only the cytoplasms of the cells is taken into account; or a "nuclear fraction", wherein the DACH2 protein expression of only the nuclei of the cells is taken into account. The nuclear fraction may for example be classified as < 2 %, 2 - 25 %, > 25 - 75 % or > 75 % immunoreactive cells of the relevant cell population. The "nuclear fraction" corresponds to the percentage of relevant cells in a sample that exhibits a positive staining in the nucleus, wherein a medium or distinct and strong immunoreactivity in the nucleus is considered positive and no or faint immunoreactivity in the nucleus is considered negative. The "cytoplasmic fraction" corresponds to the percentage of relevant cells in a sample that exhibits a positive staining in the cytoplasm, wherein a medium or distinct and strong immunoreactivity in the cytoplasm is considered positive and no or faint immunoreactivity in the cytoplasm is considered negative. The person skilled in the art of pathology understands which cells that are relevant under the conditions present when performing the method and may determine a nuclear or cytoplasmic fraction based on his general knowledge and the teachings of the present disclosure. The relevant cells may for example be tumor cells. Further, the skilled artisan understands how to perform corresponding measurements employing the "cellular fraction".

Another alternative for the quantification of DACH2 protein expression in a sample, such as the sample earlier obtained from the subject or the reference sample, is the determination of the overall staining intensity of the sample. The intensity may for example be: a "cellular intensity", wherein the DACH2 protein expression of the whole cells is taken into account; a "cytoplasmic intensity", wherein the DACH2 protein expression of only the cytoplasms of the cells is taken into account, or a "nuclear intensity", wherein the DACH2 protein expression of only the nuclei of the cells is taken into account. Nuclear intensity is subjectively evaluated in accordance with standards used in clinical histopathological diagnostics. Outcome of a nuclear intensity determination may be classified as: absent = no overall immunoreactivity in the nuclei of relevant cells of the sample, weak = faint overall immunoreactivity in the nuclei of relevant cells of the sample, moderate = medium overall immunoreactivity in the nuclei of relevant cells of the sample, or strong = distinct and strong overall immunoreactivity in the nuclei of relevant cells of the sample. Outcome of a cellular intensity determination may be classified as: absent = no overall immunoreactivity in the cytoplasms of relevant cells of the sample, weak = faint overall immunoreactivity in the cytoplasms of relevant cells of the sample, moderate = medium overall immunoreactivity in the cytoplasms of relevant cells of the sample or strong = distinct and strong overall immunoreactivity in the cytoplasms of relevant cells of the sample. In some embodiments, the weak and moderate values may be combined into a weak/moderate value. The person skilled in the art understands which cells that are relevant under the conditions present when performing the method and may determine a nuclear or cytoplasmic intensity based on his general knowledge and the teachings of the present disclosure. The relevant cells may for example be tumor cells. Further, the skilled artisan understands how to perform corresponding measurements employing the "cellular intensity".

Thus, in embodiments of the methods of the above aspects, the reference value may be a nuclear fraction, a nuclear intensity, a combination of a nuclear fraction and a nuclear intensity, a cytoplasmic fraction, a cytoplasmic intensity or a combination of a nuclear fraction and a nuclear intensity.

As indicated in the figures, more than one reference value based on expression of DACH2 protein may function as a relevant reference value for determining the level of responsiveness.

Thus, in embodiments of the methods of the above aspects, the reference value of step b) is a nuclear or cytoplasmic fraction of 95 % or lower, such as 90 % or lower, such as 85 % or lower, such as 80 % or lower, such as 75 % or lower, such as 70 % or lower, such as 65 % or lower, such as 60 % or lower, such as 55 % or lower, such as 50 % or lower, such as 45 % or lower, such as 40 % or lower, such as 35 % or lower, such as 30 % or lower, such as 25 % or lower, such as 20 % or lower, such as 15 % or lower, such as 10 % or lower, such as 5 % or lower, such as 2 % or lower, such as 1 % or lower, such as 0 %.

Further, in embodiments of the methods of the above aspects the reference value of step b) may be a moderate nuclear or cytoplasmic intensity or lower, such as a weak nuclear or cytoplasmic intensity or lower, such as an absent nuclear or cytoplasmic intensity.

Also, in embodiments of the methods of the above aspects, the reference value may be a combination or a function of a fraction value and an intensity value. The reference value may thus involve two, and even more, criteria. Examples of such a combined reference value is presented in Examples, Section 2. In embodiments of the methods of the above aspects, the reference value may thus be a "nuclear score" (NS) of 1-4, such as 2-4, such as 3.

Guided by the present disclosure, a person skilled in the art, e.g., a pathologist understands how to perform the evaluation yielding a fraction, such as a cellular, cytoplasmic or nuclear fraction, or an intensity, such as a cellular, cytoplasmic or nuclear intensity. For example, the skilled artisan may use a reference sample comprising a predetermined amount of DACH2 protein for establishing the appearance of a certain fraction or intensity.

However, a reference sample may not only be used for the provision of the actual reference value, but also for the provision of an example of a sample with an amount of DACH2 protein that is higher than the amount corresponding to the reference value. As an example, in histochemical staining, such as in immunohistochemical staining, the skilled artisan may use a reference sample for establishing the appearance of a stained sample having a high amount of DACH2 protein, e.g., a positive reference. Subsequently, the skilled artisan may assess the appearances of samples having lower amounts of DACH2 protein, such as the appearance of a sample with an amount of DACH2 protein corresponding to the reference value. In other words, the skilled artisan may use a reference sample to create a mental image of a reference value corresponding to an amount of DACH2 protein which is lower than that of the reference sample. Alternatively, or as a complement, in such assessments, the skilled artisan may use another reference sample having a low amount of DACH2 protein, or lacking detectable DACH2 protein, for establishing the appearance of such a sample, e.g., as a "negative reference".

For example, if a moderate nuclear intensity is used as the reference value, two reference samples may be employed: a first reference sample having no detectable DACH2 protein, and thus corresponding to an absent nuclear intensity, which is lower than the reference value; and a second reference sample having an amount of DACH2 protein corresponding to a strong nuclear intensity, which is higher than the reference value.

Consequently, in the evaluation, the skilled artisan may use a reference sample for establishing the appearance of a sample with a high amount of DACH2 protein. Such reference sample may be a sample comprising tissue expressing a high amount of DACH2 protein, such as a sample comprising tumor tissue having a pre-established high expression of DACH2 protein.

Accordingly, the reference sample may provide an example of a strong nuclear intensity (NI). With the knowledge of the appearance of a sample with strong NI, the skilled artisan may then divide samples into the NI categories absent, weak, moderate and strong. This division may be further assisted by a reference sample lacking detectable DACH2 protein (negative reference), i.e., a reference sample providing an absent nuclear intensity. Also, the reference sample may provide an example of a sample with a nuclear fraction (NF) higher than 75 %. With the knowledge of the appearance of a sample with more than 75 % positive cells, the skilled artisan may then evaluate the NF of other samples having e.g., a lower percentage of positive cells. This division may be further assisted by a reference sample essentially lacking DACH2 protein (negative reference), i.e., a reference sample providing a low NF (e.g., < 5%, such as < 2%), or a NF of 0. The same applies to cytoplasmic intensity and cytoplasmic fraction.

As mentioned above, cell lines expressing a controlled amount of DACH2 protein may be used as the reference, in particular as a positive reference.

One or more pictures may also be provided as the "reference sample". For example, such a picture may show an example of a tumor tissue slide stained with a certain antibody during certain conditions and exhibiting a certain nuclear intensity and/or fraction. The above discussion about the "reference sample" applies *mutatis mutandis* to pictures.

The cell lines or pictures may also form part of the kit according to the present disclosure (see below).

As shown in the figures, both DACH2 mRNA (figure 6) and DACH2 protein (figure 5) is expressed to higher degree in cisplatin resistant cells than in cisplatin sensitive cells. Accordingly, the "level of DACH2" refers to the level of DACH2 protein or the level of DACH2 mRNA in the context of the present disclosure. In the Examples below, the DACH2 protein expression is measured and shown to be significantly correlated with outcome of platinum-based treatment (see also Figure 1-4). Thus, in preferred embodiments, "DACH2" refers to DACH2 protein.

The skilled person should recognize that the usefulness of the methods according to the above aspects is not limited to the quantification of any particular variant of the DACH2 protein or DACH2 mRNA present in the subject in question, as long as the protein is encoded by the relevant gene and presents the relevant pattern of expression. There are several isoforms/splice variants of DACH2, of which four are represented by SEQ ID NO:2-5. SEQ ID NO:1 represents a DACH2 subsequence that is common to SEQ ID NO:2, 3 and 4, while SEQ ID NO:5 includes the whole of SEQ ID NO:1 but five N-terminal amino acids.

As a non-limiting example, the DACH2 protein may comprise a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the DACH2 protein may comprise, or consists of, a sequence selected from:
i) SEQ ID NO:2, 3, 4 or 5; and
ii) a sequence which is at least 85 % identical to SEQ ID NO:2, 3, 4 or 5.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2, 3, 4 or 5.

The term "% identical", as used in the context of the present disclosure, is calculated as follows. The query sequence is aligned to the target sequence using the CLUSTAL W algorithm (Thompson, J.D., Higgins, D.G. and Gibson, T.J., Nucleic Acids Research, 22: 4673-4680 (1994)). The amino acid residues at each position are compared, and the percentage of positions in the query sequence that have identical correspondences in the target sequence is reported as % identical. Also, the target sequence determines the number of positions that are compared. Consequently, in the context of the present disclosure, a query sequence that is shorter than the target sequence can never be 100 % identical to the target sequence. For example, a query sequence of 85 amino acid residues may at the most be 85 % identical to a target sequence of 100 amino acid residues.

In some embodiments, step a) of the methods of the above aspects may comprise:
obtaining biological material from the subject, excising or selecting a relevant part of the biological material to obtain said sample and optionally arranging the sample on a solid phase to facilitate the evaluation of step a). Step a) may thus, as an example, comprise obtaining tumor tissue material from the subject, optionally fixating the tissue material in paraffin or formalin, histo-processing the tissue material to obtain a section which constitute said sample and optionally mounting said sample on a transparent slide, such as a glass slide, for microscopy.

In embodiments of the methods of the aspects above, the DACH2 protein may be detected and/or quantified through the application to the sample of a detectable and/or quantifiable affinity ligand, which is capable of selective interaction with the DACH2 protein. The application of the affinity ligand is performed under conditions that enable binding of the affinity ligand to DACH2 protein in the sample.

To concretize, in embodiments of the methods of the aspects above, step a) may comprise:
a1) applying to said sample a quantifiable affinity ligand capable of selective interaction with the DACH2 protein to be evaluated, said application being performed under conditions that enable binding of said affinity ligand to DACH2 protein present in said sample;
a2) removing non-bound affinity ligand; and
a3) quantifying the affinity ligand remaining in association with said sample to evaluate said amount.

"Affinity ligand remaining in association with the sample" refers to affinity ligand which was not removed in step a2), e.g., the affinity ligand bound to the sample. Here, the binding may for example be the interaction between antibody and antigen.

However, in some embodiments, the removal of non-bound affinity ligand according to a2), e.g. the washing, is not always necessary. Thus, in some embodiments of the methods of the aspects above, step a) may comprise:
aI) applying to said sample a quantifiable affinity ligand capable of selective interaction with the DACH2 protein to be evaluated, said application being performed under conditions that enable binding of said affinity ligand to DACH2 protein present in said sample;
aII) quantifying the affinity bound to said sample to evaluate said amount.

In the context of the present disclosure, "specific" or "selective" interaction of e.g., an affinity ligand with its target or antigen means that the interaction is such that a distinction between specific and non-specific, or between selective and non-selective, interaction becomes meaningful. The interaction between two proteins is sometimes measured by the dissociation constant. The dissociation constant describes the strength of binding (or affinity) between two molecules. Typically the dissociation constant between an antibody and its antigen is from 10⁻⁷ to 10⁻¹¹ M. However, high specificity/selectivity does not necessarily require high affinity. Molecules with low affinity (in the molar range) for its counterpart have been shown to be as selective/specific as molecules with much higher affinity. In the case of the present disclosure, a specific or selective interaction refers to the extent to which a particular method can be used to determine the presence and/or amount of a specific protein, the target protein, under given conditions in the presence of other proteins in a sample of biological origin, such as an prepared tissue sample or fluid sample of a naturally occurring or processed biological fluid. In other words, specificity or selectivity is the capacity to distinguish between related proteins. Specific and selective are sometimes used interchangeably in the present description. For example, the specificity or selectivity of an antibody may be determined using a protein array set-up, a suspension bead array and a multiplexed competition assay, respectively (see e.g. Nilsson P et al. (2005) Proteomics 5:4327-4337 Specificity, which describes specificity and selectivity determinations).

It is regarded as within the capabilities of those of ordinary skill in the art to select or manufacture the proper affinity ligand and to select the proper format and conditions for detection and/or quantification. Nevertheless, examples of affinity ligands that may prove useful, as well as examples of formats and conditions for detection and/or quantification, are given below.

Thus, in embodiments of the present disclosure, the affinity ligand may be selected from the group consisting of antibodies, fragments thereof and derivatives thereof, i.e., affinity ligands based on an immunoglobulin scaffold. The antibodies and the fragments or derivatives thereof may be isolated. Antibodies comprise monoclonal and polyclonal antibodies of any origin, including murine, rabbit, human and other antibodies, as well as chimeric antibodies comprising sequences from different species, such as partly humanized antibodies, e.g., partly humanized mouse antibodies. Polyclonal antibodies are produced by immunization of animals with the antigen of choice. The polyclonal antibodies may be antigen purified. Monoclonal antibodies of defined specificity can be produced using the hybridoma technology developed by Köhler and Milstein (Köhler G and Milstein C (1976) Eur. J. Immunol. 6:511-519). The antibody fragments and derivatives of the present disclosure are capable of selective interaction with the same antigen (e.g. DACH2 protein) as the antibody they are fragments or derivatives of. Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137). They further comprise Bence Jones dimers (Stevens FJ et al. (1991) Biochemistry 30:6803-6805); camelid heavy-chain dimers (Hamers-Casterman C et al. (1993) Nature 363:446-448) and single variable domains (Cai X and Garen A (1996) Proc. Natl. Acad. Sci. U.S.A. 93:6280-6285; Masat L et al. (1994) Proc. Natl. Acad. Sci. U.S.A. 91:893-896), and single domain scaffolds like e.g., the New Antigen Receptor (NAR) from the nurse shark (Dooley H et al. (2003) Mol. Immunol. 40:25-33) and minibodies based on a variable heavy domain (Skerra A and Plückthun A (1988) Science 240:1038-1041).

In some embodiments, the affinity ligand of the present disclosure is capable of selective interaction with a peptide consisting of the amino acid sequence SEQ ID NO:1. The DACH2 fragment SEQ ID NO: was designed to lack transmembrane regions to ensure efficient expression in *E. coli*, and to lack any signal peptide, since those are cleaved off in the mature protein. SEQ ID NO:1 was thus designed for immunizations. In addition, the protein fragment was designed to consist of a unique sequence with low sequence identity to other human proteins, to minimize cross reactivity of generated affinity reagents, and to be of a suitable size to allow the formation of conformational epitopes and still allow efficient cloning and expression in bacterial systems. Accordingly, in the cases wherein the affinity ligand is an antibody or fragment o derivative thereof, the affinity ligand may be obtainable by a process comprising a step of immunizing an animal with a peptide whose amino acid sequence consists of the sequence SEQ ID NO:1. For example, an immunization process may comprise primary immunization with the protein/antigen in Freund's complete adjuvant. Also, the immunization process may further comprise boosting at least two times, in intervals of 2-6 weeks, with the protein/antigen in Freund's incomplete adjuvant. Processes for the production of antibodies or fragments or derivatives thereof against a given target are known in the art.

In the context of the present disclosure, an "antigen purified antibody" is one or a population of polyclonal antibodies which has been affinity purified on its own antigen, thereby separating such antigen purified antibodies from other antiserum proteins and non-specific antibodies. This affinity purification results in antibodies that bind selectively to its antigen. The polyclonal antisera may for example be purified by a two-step immunoaffinity-based protocol to obtain antigen purified antibodies selective for the target protein. Antibodies directed against generic affinity tags of antigen fragments are removed in a primary depletion step, using the immobilized tag protein as the capturing agent. Following the first depletion step, the serum is loaded on a second affinity column with the antigen as capturing agent, in order to enrich for antibodies specific for the antigen (see also Nilsson P et al. (2005) Proteomics 5:4327-4337).

Polyclonal and monoclonal antibodies, as well as their fragments and derivatives, represent the traditional choice of affinity ligands in applications requiring selective biomolecular recognition, such as in the detection and/or quantification of DACH2 protein according to the method aspects above. However, those of skill in the art know that, due to the increasing demand of high throughput generation of selective binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific/selective affinity ligands are then selected using a suitable selection platform. The scaffold molecule may be of immunoglobulin protein origin (Bradbury AR and Marks JD (2004) J. Immunol. Meths. 290:29-49), of non-immunoglobulin protein origin (Nygren PÅ and Skerra A (2004) J. Immunol. Meths. 290:3-28), or of an oligonucleotide origin (Gold L et al. (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against DACH2 protein for use according to the present disclosure, are staphylococcal protein A and domains thereof and derivatives of these domains, such as protein Z (Nord K et al. (1997) Nat. Biotechnol. 15:772-777); lipocalins (Beste G et al. (1999) Proc. Natl. Acad. Sci. U.S.A. 96:1898-1903); ankyrin repeat domains (Binz HK et al. (2003) J. Mol. Biol. 332:489-503); cellulose binding domains (CBD) (Smith GP et al. (1998) J. Mol. Biol. 277:317-332; Lehtiö J et al. (2000) Proteins 41:316-322); γ crystallines (Fiedler U and Rudolph R, WO01/04144); green fluorescent protein (GFP) (Peelle B et al. (2001) Chem. Biol. 8:521-534); human cytotoxic T lymphocyte-associated antigen 4 (CTLA-4) (Hufton SE et al. (2000) FEBS Lett. 475:225-231; Irving RA et al. (2001) J. Immunol. Meth. 248:31-45); protease inhibitors, such as Knottin proteins (Wentzel A et al. (2001) J. Bacteriol. 183:7273-7284; Baggio R et al. (2002) J. Mol. Recognit. 15:126-134) and Kunitz domains (Roberts BL et al. (1992) Gene 121:9-15; Dennis MS and Lazarus RA (1994) J. Biol. Chem. 269:22137-22144); PDZ domains (Schneider S et al. (1999) Nat. Biotechnol. 17:170-175); peptide aptamers, such as thioredoxin (Lu Z et al. (1995) Biotechnology 13:366-372; Klevenz B et al. (2002) Cell. Mol. Life Sci. 59:1993-1998); staphylococcal nuclease (Norman TC et al. (1999) Science 285:591-595); tendamistats (McConell SJ and Hoess RH (1995) J. Mol. Biol. 250:460-479; Li R et al. (2003) Protein Eng. 16:65-72); trinectins based on the fibronectin type III domain (Koide A et al. (1998) J. Mol. Biol. 284:1141-1151*;* Xu L et al. (2002) Chem. Biol. 9:933-942); and zinc fingers (Bianchi E et al. (1995) J. Mol. Biol. 247:154-160; Klug A (1999) J. Mol. Biol. 293:215-218; Segal DJ et al. (2003) Biochemistry 42:2137-2148).

The above-mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two-hybrid system (Fields S and Song O (1989) Nature 340:245-246), yeast display (Gai SA and Wittrup KD (2007) Curr Opin Struct Biol 17:467-473), mRNA display (Roberts RW and Szostak JW (1997) Proc. Natl. Acad. Sci. U.S.A. 94:12297-12302), bacterial display (Daugherty PS (2007) Curr Opin Struct Biol 17:474-480, Kronqvist N et al. (2008) Protein Eng Des Sel 1-9, Harvey BR et al. (2004) PNAS 101 (25):913-9198), microbead display (Nord O et al. (2003) J Biotechnol 106:1-13, WO01/05808), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the present disclosure, the affinity ligand may be a non-immunoglobulin affinity ligand derived from any of the protein scaffolds listed above, or an oligonucleotide molecule.

As mentioned above, the DACH2 protein fragment SEQ ID NO:1 was designed to consist of a unique sequence with low sequence identity to other human proteins and to minimize cross reactivity of generated affinity reagents. Also, SEQ ID NO:1 represents a region common to many DACH2 splice variants. Consequently, in embodiments of the present disclosure, the affinity ligand may be capable of selective interaction with a polypeptide consisting of the amino acid sequence SEQ ID NO:1.

The detection and/or quantification of the affinity ligand capable of selective interaction with the DACH2 protein may be accomplished in any way known to the skilled person for detection and/or quantification of binding reagents in assays based on biological interactions. Accordingly, any affinity ligand described above may be used to quantitatively and/or qualitatively detect the presence of the DACH2 protein. These "primary" affinity ligands may be labeled themselves with various markers or may in turn be detected by secondary, labeled affinity ligands to allow detection, visualization and/or quantification. This can be accomplished using any one or more of a multitude of labels, which can be conjugated to the affinity ligand capable of interaction with DACH2 protein or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g., fluorescein, rhodamine, phycoerythrin, fluorescamine), chromophoric dyes (e.g., rhodopsin), chemiluminescent compounds (e.g., luminal, imidazole) and bioluminescent proteins (e.g., luciferin, luciferase), haptens (e.g., biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g., horseradish peroxidase, alkaline phosphatase, beta-lactamase), radioisotopes (e.g., ³H, ¹⁴C, ³²P, ³⁵S or ¹²⁵I) and particles (e.g., gold). In the context of the present disclosure, "particles" refer to particles, such as metal particles, suitable for labeling of molecules. Such particles are well known to the skilled person. Further, the affinity ligands may also be labeled with fluorescent semiconductor nanocrystals (quantum dots). Quantum dots have superior quantum yield and are more photostable compared to organic fluorophores and are therefore more easily detected (Chan et al. (2002) Curr Opi Biotech. 13: 40-46). The different types of labels can be conjugated to an affinity ligand using various chemistries, e.g., the amine reaction or the thiol reaction. However, other reactive groups than amines and thiols can be used, e.g., aldehydes, carboxylic acids and glutamine.

The method aspects above may be put to use in any of several known formats and set-ups, of which a non-limiting selection is discussed below.

In a set-up based on histology, the detection, localization and/or quantification of a labeled affinity ligand bound to its DACH2 protein target may involve visualizing techniques, such as light microscopy or immunofluoresence microscopy. Other methods may involve the detection via flow cytometry or luminometry.

Biological material from the subject may be used for obtaining the sample for detection and/or quantification of DACH2 protein. The sample may thus be an earlier obtained sample. If using an earlier obtained sample in a method, no steps of the method are practiced on the human or animal body.

The affinity ligand may be applied to the sample for detection and/or quantification of the DACH2 protein. This procedure enables not only detection of DACH2 protein, but may in addition show the distribution and relative level of expression thereof.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light in a specific wavelength region. The presence of a luminescently tagged affinity ligand may be detected and/or quantified by luminescence developed during a chemical reaction. Detection of an enzymatic reaction is due to a color shift in the sample arising from a chemical reaction. Those of skill in the art are aware that a variety of different protocols can be modified in order for proper detection and/or quantification.

In embodiments of the methods of the above aspects, the sample may be immobilized onto a solid phase support or carrier, such as nitrocellulose or any other solid support matrix capable of immobilizing DACH2 protein present in the biological sample applied to it. Some well-known solid state support materials useful in the present invention include glass, carbohydrate (e.g., Sepharose), nylon, plastic, wool, polystyrene, polyethene, polypropylene, dextran, amylase, films, resins, cellulose, polyacrylamide, agarose, alumina, gabbros and magnetite. After immobilization of the biological sample, primary affinity ligand selective for DACH2 protein may be applied. If the primary affinity ligand is not labeled in itself, the supporting matrix may be washed with one or more appropriate buffers known in the art, followed by exposure to a secondary labeled affinity ligand and washed once again with buffers to remove unbound affinity ligands. Thereafter, selective affinity ligands may be detected and/or quantified with conventional methods. The binding properties for an affinity ligand may vary from one solid state support to the other, but those skilled in the art should be able to determine operative and optimal assay conditions for each determination by routine experimentation.

Consequently, in embodiments of the methods of the above aspects, the quantifiable affinity ligand of a1) or aI) may be detected using a secondary affinity ligand capable of recognizing the quantifiable affinity ligand. The quantification of a3) or aII) may thus be carried out by means of a secondary affinity ligand with affinity for the quantifiable affinity ligand. As an example, the secondary affinity ligand may be an antibody or a fragment or a derivative thereof.

As an example, one available method for detection and/or quantification of the DACH2 protein is by linking the affinity ligand to an enzyme that can then later be detected and/or quantified in an enzyme immunoassay (such as an EIA or ELISA). Such techniques are well established, and their realization does not present any undue difficulties to the skilled person. In such methods, the biological sample is brought into contact with a solid material or with a solid material conjugated to an affinity ligand against the DACH2 protein, which is then detected and/or quantified with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the present disclosure are ³H, ¹⁴C ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize DACH2 protein by detection of radioactivity *in vivo* or *ex vivo.* Radionuclear scanning with e.g., gamma camera, magnetic resonance spectroscopy or emission tomography function for detection *in vivo* and *ex vivo,* while gamma/beta counters, scintillation counters and radiographies are also used *ex vivo.*

Methods for detecting and quantifying biomarkers on the mRNA level are well known within the art. Further, one example of such a method is described below under Examples.

In general, total cellular RNA may be purified from cells by homogenization in the presence of nucleic acid extraction buffer, followed by centrifugation. Nucleic acids are then precipitated, in order to remove DNA by treatment with DNase and precipitation. The RNA molecules are then separated by gel electrophoresis on agarose gels according to standard techniques, and transferred to nitrocellulose filters by, e.g., the so-called "Northern" blotting technique. The RNA is then immobilized on the filters by heating. Detection and quantification of specific RNA is accomplished using appropriately labeled DNA or RNA probes complementary to the RNA in question. See, for example, Molecular Cloning: A Laboratory Manual (Sambrook J.et al., (1989) 2nd edition, Cold Spring Harbor Laboratory Press). Methods for the preparation of labeled DNA and RNA probes, and the conditions for hybridization thereof to target nucleotide sequences, are described in Molecular Cloning: A Laboratory Manual (Sambrook J.et al., (1989) 2nd edition, Cold Spring Harbor Laboratory Press). For example, the nucleic acid probe may be labeled with, e.g., a radionuclide such as ³H, ³²P, ³³P ¹⁴C or ³⁵S; a heavy metal; or a ligand capable of functioning as a specific binding pair member for a labeled ligand (e.g., biotin, avidin, or an antibody), a fluorescent molecule, a chemi luminescent molecule, an enzyme, or the like.

Probes may be labeled to high specific activity by either the nick translation method (Rigby et al., (1977) J. Mol Biol, 113: 237-251), or by the random priming method (Fienberg, (1983) Anal. Biochem., 132: 6-13). The latter can be a method for synthesizing ³²P-labeled probes of high specific activity from RNA templates. For example, by replacing preexisting nucleotides with highly radioactive nucleotides according to the nick translation method, it is possible to prepare ³²P- labeled nucleic acid probes with a specific activity well in excess of 10 cpm/microgram. Autoradiographic detection of hybridization then can be performed by exposing hybridized filters to photographic film. Densitometric scanning of the photographic films exposed by the hybridized filters provides an accurate measurement of biomarker levels. Using another approach, biomarker levels can be quantified by computerized imaging systems, such as the Molecular Dynamics 400-B 2D Phosphorimager (Amersham Biosciences, Piscataway, NJ., USA).

Where radionuclide labeling of DNA or RNA probes is not practical, the random- primer method can be used to incorporate an analogue, for example, the dTTP analogue 5 -(N-(N- biotinyl-epsilon-aminocaproyl)-3-aminoallyl)deoxyuridine triphosphate, into the probe molecule. The biotinylated probe oligonucleotide can be detected by reaction with biotin-binding proteins, such as avidin, streptavidin, and antibodies (e.g., anti-biotin antibodies) coupled to fluorescent dyes or enzymes that produce color reactions.

In addition to Northern and other RNA blotting hybridization techniques, determining the levels of RNA transcript may be accomplished using the technique of in situ hybridization. This technique requires fewer cells than the Northern blotting technique, and involves depositing whole cells onto a microscope cover slip and probing the nucleic acid content of the cell with a solution containing radioactive or otherwise labeled nucleic acid (e.g., cDNA or RNA) probes. This technique is particularly well-suited for analyzing tissue biopsy samples from subjects.

The relative number of RNA transcripts in cells also can be determined by reverse transcription of RNA transcripts, followed by amplification of the reverse-transcribed transcripts by polymerase chain reaction (RT-PCR). The levels of RNA transcripts can be quantified in comparison with an internal standard, for example, the level of mRNA from a standard gene present in the same sample. The person skilled in the art is capable of selecting suitable genes for use as an internal standard. The methods for quantitative RT-PCR and variations thereof are within the skill in the art.

Any suitable primers can be used for the quantitative RT-PCR. Preferably, the primers are specific to DACH2. It is within the skill in the art to generate primers specific to DACH2. Primers can be of any suitable length, but are preferably between 19 and 23 (e.g., 19, 20, 21, 22, or 23) nucleotides. Ideally, amplicon length should be 50 to 150 (up to 250 may be necessary but then optimization of the thermal cycling protocol and reaction components may be necessary) bases for optimal PCR efficiency. Designing primers that generate a very long amplicon may lead to poor amplification efficiency. Information about primer design and optimal amplicon size may fo example be found at www.ambion.com.

In some instances, it may be desirable to use microchip technology to detect biomarker expression. The microchip can be fabricated by techniques known in the art. For example, probe oligonucleotides of an appropriate length, e.g., 40 nucleotides, are 5'-amine modified at position C6 and printed using commercially available microarray systems, e.g., the GENEMACHINE OmniGrid 100 Microarrayer and Amersham CODELINK activated slides. Labeled cDNA oligomer corresponding to the target RNAs is prepared by reverse transcribing the target RNA with labeled primer. Following first strand synthesis, the RNA/DNA hybrids are denatured to degrade the RNA templates. The labeled target cDNAs thus prepared are then hybridized to the microarray chip under hybridizing conditions, e.g., 6 times SSPE/30% formamide at 25 °C for 18 hours, followed by washing in 0.75 times TNT at 37 °C for 40 minutes. At positions on the array, where the immobilized probe DNA recognizes a complementary target cDNA in the sample, hybridization occurs. The labeled target cDNA marks the exact position on the array where binding occurs, thereby allowing automatic detection and quantification. The output consists of a list of hybridization events, which indicate the relative abundance of specific cDNA sequences, and therefore the relative abundance of the corresponding complementary biomarker, in the subject sample. According to one embodiment, the labeled cDNA oligomer is a biotin-labeled cDNA prepared from a biotin-labeled primer. The microarray is then processed by direct detection of the biotin-containing transcripts using, e.g., Streptavidin-Alexa647 conjugate, and scanned utilizing conventional scanning methods. Image intensities of each spot on the array are proportional to the abundance of the corresponding biomarker in the subject sample.

The use of the array has one or more advantages for mRNA expression detection. First, the global expression of several to thousands of genes can be identified in a single sample at one time. Second, through careful design of the oligonucleotide probes, the expression of both mature and precursor molecules can be identified. Third, in comparison with Northern blot analysis, the chip requires a small amount of RNA.

The DACH2 mRNA may for example be extracted from formalin-fixed, paraffin-embedded tumor tissue. Accordingly, the sample of the methods of the present disclosure may be formalin-fixed and/or paraffin-embedded prostate tumor tissue independent of if DACH2 protein or DACH2 mRNA is detected.

The inventors have realized that DACH2 mRNA analysis of the present disclosure may be incorporated in an mRNA-based assay designed to support individualized treatment planning. Such an assay may employ RT-PCR to analyze the expression of several genes.

As a fourth aspect of the present disclosure, there is provided a kit for carrying out a method according to one or more of the above aspects, which comprises:
a) a quantifiable affinity ligand capable of selective interaction with a DACH2 protein; and
b) reagents necessary for quantifying the amount of said quantifiable affinity ligand.

Various components of the kit according to the fourth aspect may be selected and specified as described above in connection with the method aspects of the present disclosure.

Thus, the kit according to the present disclosure comprises an affinity ligand against a DACH2 protein, as well as other means that help to quantify the specific and/or selective affinity ligand after it has bound specifically and/or selectively to the DACH2 protein. For example, the kit may contain a secondary affinity ligand for detecting and/or quantifying a complex formed by the DACH2 protein and the affinity ligand capable of selective interaction with the DACH2 protein. The kit may also contain various auxiliary substances other than affinity ligands, to enable the kit to be used easily and efficiently. Examples of auxiliary substances include solvents for dissolving or reconstituting lyophilized protein components of the kit, wash buffers, substrates for measuring enzyme activity in cases where an enzyme is used as a label, target retrieval solution to enhance the accessibility to antigens in cases where paraffin or formalin-fixed tissue samples are used, and substances such as reaction arresters, e.g., endogenous enzyme block solution to decrease the background staining and/or counterstaining solution to increase staining contrast, that are commonly used in immunoassay reagent kits.

In embodiments of the kit aspect, the affinity ligand may be any one of the affinity ligands described above in connection with the method aspects.

Further, in accordance with what is described above in connection with the method aspects, the detectable affinity ligand may in embodiments of the kit aspect comprise a label selected from the group consisting of fluorescent dyes and metals, chromophoric dyes, chemiluminescent compounds and bioluminescent proteins, enzymes, radioisotopes, particles and quantum dots. Alternatively, the reagents necessary for quantifying the amount of the affinity ligand comprise a secondary affinity ligand capable of recognizing the quantifiable affinity ligand. As an example, the secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes, particles and quantum dots.

The kit according to the kit aspect may also advantageously comprise a reference sample for provision of, or yielding, the reference value to be used for comparison with the sample value. For example, the reference sample may comprise a predetermined amount of DACH2 protein. Such a reference sample may for example be constituted by a tissue sample containing the predetermined amount of DACH2 protein. The tissue reference sample may then be used by the person of skill in the art in the determination of the DACH2 expression status in the sample being studied, by manual, such as ocular, or automated comparison of expression levels in the reference tissue sample and the subject sample. As another example, the reference sample may comprise cell lines, such as cancer cell lines, expressing a predetermined, or controlled, amount of DACH2 protein. As an example, the cell lines may be formalin fixed. Also, such formalin fixed cell lines may be paraffin embedded.

The wording "reference sample for provision of the reference value" is to be interpreted broadly in the context of the present disclosure. The reference sample may comprise an amount of DACH2 protein actually corresponding to the reference value, but it may also comprise an amount of DACH2 protein corresponding to a value being higher than the reference value. In the latter case, the "high" value may be used by a person performing the method as an upper reference (positive reference) for assessing, e.g., the appearance of, a reference value that is lower than the "high" value. The person skilled in the art of immunohistochemistry understands how to do such an assessment. Further, as an alternative or a complementing example, the skilled person may use another reference sample comprising a low amount of DACH2 protein for provision of a "low" value in such an assessment, e.g., as a negative reference. This is further discussed above in connection with the method aspects.

Consequently, in embodiments of the kit aspect, the reference sample may comprise an amount of DACH2 protein corresponding to the reference value. As an example, the reference sample may comprise an amount of DACH2 protein corresponding to a nuclear or cytoplasmic fraction of 95 % or lower, such as 90 % or lower, such as 85 % or lower, such as 80 % or lower, such as 75 % or lower, such as 70 % or lower, such as 65 % or lower, such as 60 % or lower, such as 55 % or lower, such as 50 % or lower, such as 45 % or lower, such as 40 % or lower, such as 35 % or lower, such as 30 % or lower, such as 25 % or lower, such as 20 % or lower, such as 15 % or lower, such as 10 % or lower, such as 5 % or lower, such as 2 % or lower, such as 1 % or lower, such as 0 %.

Alternatively, or as a complement, the reference sample may comprise an amount of DACH2 protein corresponding to a moderate nuclear or cytoplasmic intensity or lower, such as a weak nuclear or cytoplasmic intensity or lower, such as an absent nuclear or cytoplasmic intensity.

The provision of fraction values and intensity values is discussed above in connection with the method aspects.

Further, in alternative or complementing embodiments of the kit aspect, the kit may comprise a reference sample comprising an amount of DACH2 protein corresponding to a value being higher than the reference value. In these embodiments, the reference sample may for example comprise an amount of DACH2 protein corresponding to a nuclear or cytoplasmic fraction of 75 % or higher and/or a strong nuclear or cytoplasmic intensity.

In yet further alternative or complementing embodiments of the kit aspect, the kit may comprise a reference sample comprising an amount of DACH2 protein corresponding to a value being lower than or equal to the reference value, e.g., an absent nuclear or cytoplasmic intensity and/or a nuclear or cytoplasmic fraction of < 2 %, such as 0 %.

The kit may thus comprise: a reference sample comprising an amount of DACH2 protein corresponding to a predetermined reference value; a reference sample comprising an amount of DACH2 protein corresponding to a value being higher than a predetermined reference value; and/or a reference sample comprising an amount of DACH2 protein corresponding to a value being lower than or equal to a predetermined reference value.

In embodiments of the kit aspect, the reference sample may be a tissue sample, such as a tissue sample adapted to ocular or microscopic evaluation. As an example, the tissue reference sample may be fixated in paraffin or buffered formalin and/or histo-processed to sections (e.g., µm-thin sections) that are mounted on microscopic glass-slides. The tissue reference sample may be further adapted to staining with affinity ligands, such as antibodies, against a DACH2 protein.

Consequently, in embodiments of the kit aspect, the reference sample may be adapted to directly, or indirectly, provide any relevant reference value, such as any one of the reference values discussed above.

Further embodiments of the reference sample of the kit aspect are discussed above in connection with the reference values and reference samples of the method aspects.

Following the findings presented above, the inventors have realized several uses for the DACH2 protein and fragments thereof.

Thus, as a fifth aspect of the present disclosure, there is provided a DACH2 protein fragment which consists of 200 amino acids, such as 150 amino acids or less and comprises SEQ ID NO:1. As a configuration of the fifth aspect, there is provided a polypeptide fragment of SEQ ID NO:1 which consists of 15-118 amino acids, such as 30-100 amino acids.

Possible uses of such fragments are described below.

As a first configuration of a sixth aspect of the present disclosure, there is provided a use of DACH2 (i.e. a DACH2 protein or DACH2 mRNA) as a treatment predictive marker for platinum-based treatment of a mammalian subject having a cancer.

The use of the first configuration may be entirely *ex vivo,* e.g., on previously obtained samples.

In the context of the present disclosure, "treatment predictive marker" refers to something material which presence indicates a level of responsiveness to a treatment. The marker may thus be a biomarker, such as a human protein. It is to be understood from the above that the presence of the treatment predictive marker, or a relatively high level thereof, is indicative of a relatively low responsiveness to the treatment, while the absence of the treatment predictive marker, or a relatively low level thereof, is indicative of a relatively high responsiveness to the treatment. Here, the "relatively high responsiveness" is high in comparison with the "relatively low responsiveness".

As a second configuration of the sixth aspect, there is provided a use of a DACH2 protein or an antigenically active fragment thereof for the production, selection or purification of a treatment predictive agent for platinum-based treatment of a mammalian subject having a cancer.

The selection and purification may be *ex vivo,* while the production may be *in vivo* (normally in an animal).

An "antigenically active fragment" refers to a fragment of sufficient size to be capable of generating an affinity ligand capable of selective interaction with the fragment.

In the context of the present disclosure, "treatment predictive agent" refers to an agent having at least one property being valuable in an establishment of a treatment prediction. For example, the treatment predictive agent may be capable of selective interaction with the treatment predictive marker. In the context of immunohistochemistry, a relatively dense staining with the treatment predictive agent of the present disclosure is thus indicative of a relatively low responsiveness to the platinum-based treatement.

The treatment predictive agent may be an affinity ligand capable of selective interaction with the DACH2 protein or the antigenically active fragment thereof. Examples of such affinity ligands are discussed above in connection with the method aspects.

Guided by the teachings of the present disclosure, the person skilled in the art understands how to use the DACH2 protein or fragment in the production, selection or purification of the treatment predictive agent. For example, the use may comprise affinity purification on a solid support onto which the DACH2 protein or fragment thereof has been immobilized. The solid support may for example be arranged in a column. Further, the use may comprise selection of affinity ligands having specificity for the DACH2 protein or fragment thereof using a solid support onto which the DACH2 protein or fragment thereof has been immobilized. Such solid support may be well plates (such as 96 well plates), magnetic beads, agarose beads or sepharose beads. Further, the use may comprise analysis of affinity ligands on a soluble matrix, for example using a dextran matrix, or use in a surface plasmon resonance instrument, such as a Biacore™ instrument, wherein the analysis may for example comprise monitoring the affinity of a number of potential affinity ligands for the immobilized DACH2 protein or fragment thereof.

Also, for the production of the treatment predictive agent, the DACH2 protein or an antigenically active fragment thereof may be used in an immunization of an animal, such as a rabbit or mouse.

Such use may be involved in a method comprising the steps:
i) immunizing an animal using the DACH2 protein or antigenically an active fragment thereof as the antigen;
ii) obtaining serum comprising the treatment predictive agent from the immunized animal; and, optionally,
iii) isolating the treatment predictive agent from the serum. Alternatively the steps following the first step may be:
ii') obtaining cells from the immunized animal, which cells comprise DNA encoding the treatment predictive agent,
iii') fusing the cells with myeloma cells to obtain at least one clone, and
iv') obtaining the treatment predictive agent expressed by the clone.

In embodiments of the sixth aspect, the amino acid sequence of the DACH2 protein may comprise a sequence selected from:
i) SEQ ID NO:1; and
ii) a sequence which is at least 85 % identical to SEQ ID NO:1.

In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

Further, in embodiments of the sixth aspect the amino acid sequence of the DACH2 protein may comprise or consist of a sequence selected from:
i) SEQ ID NO:2, 3, 4 or 5; and
ii) a sequence which is at least 85 % identical to SEQ ID NO:2, 3, 4 or 5.

In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2, 3, 4 or 5.

The antigenically active fragment of the sixth aspect may for example be any one of the fragments of the fifth aspect.

As a seventh aspect of the present disclosure, there is provided an affinity ligand capable of selective interaction with a DACH2 protein.

Different embodiments of such an affinity ligand are discussed above in connection with the method aspects.

The affinity ligand of the seventh aspect may for example be provided as a staining solution in a container adapted for an automatic staining apparatus, such as BenchMark XT or BenchMark Ultra marketed by Ventana. The container may thus be connectable to an inlet for solutions of primary antibodies provided on the apparatus.

As an eighth aspect of the present disclosure, there is provided a use of an affinity ligand according to the seventh aspect as a treatment predictive agent for platinum-based treatment of a mammalian subject having a cancer.

Similarly, there is provided a use of an affinity ligand capable of selective interaction with a DACH2 protein for indicating whether a mammalian subject having a cancer should be given a platinum-based treatment.

Here, "should be given a platinum-based treatment" refers to the case where the benefits of the treatment overcome the disadvantages of the same. The benefits of the treatment refers to a higher probability of survival or recovery if undergoing the treatment than if not undergoing the treatment. The disadvantages of the treatment refer to side-effects, pain or other inconveniences and costs.

Such uses may for example be performed *ex vivo,* e.g., involving the determination of the amount of DACH2 in at least part of a sample earlier obtained from the subject.

Also, there is provided a use of an affinity ligand capable of selective interaction with a DACH2 protein in the manufacture of a treatment predictive agent for platinum-based treatment of a mammalian subject having a cancer.

In the present disclosure, platinum-based treatment is shown to be particularly beneficial for those subjects who are DACH2 low. The cancer subjects who are DACH2 low are a previously unrecognized subgroup in the context of platinum-based treatment.

Thus, as a ninth aspect of the present disclosure, there is provided a platinum-based therapeutic agent for use in treatment of a mammalian subject having a cancer, wherein said subject is diagnosed as DACH2 low.

As a configuration of the ninth aspect of the present disclosure, there is provided a use of a platinum-based therapeutic agent in the manufacture of a medicament for treatment of a mammalian subject having a cancer, wherein said subject is diagnosed as DACH2 low.

The subject is "DACH2 low" if any DACH2 protein or mRNA parameter derived from said subject indicates a relatively high responsiveness to platinum-based treatment. For example, the subject may be considered DACH2 low if a relevant biological sample from the subject has been found to contain an amount of DACH2 corresponding to a sample value being lower than or equal to a relevant reference value. Relevant sample and reference values are discussed above in connection with the method aspects. Thus, the cancer subject may for example be considered DACH2 low if a relevant sample, such as a tissue sample from a tumor, shows no detectable DACH2 protein expression in relevant parts of the sample, such in the tumor cells. Further, the cancer subject may for example be considered DACH2 protein low if such sample contains an amount of DACH2 protein corresponding to a nuclear score which is lower than or equal to 3 or a nuclear fraction which is lower than or equal to 25 %. From the present disclosure, the person skilled in the art, such as a pathologist, understands how to determine whether the subject is DACH2 low or not.

Various embodiments of the ninth aspect, in particular with regard to the type of cancer and the type of platinum-based therapeutic agent, are discussed above in connection with the method aspects.

In general, the inventors believe that the DACH2 protein expression is more relevant than the DACH2 mRNA expression in the context of the present disclosure.

### Examples

### 1. DACH2 expression in various cancer types

Table 1 shows the level of DACH2 protein expression in tissue samples from various cancer types. Up to 12 samples for each cancer type was tested. DACH2 protein expression was found in all cancer types tested but head and neck cancer, malignant lymphoma and lung cancer. However, samples from only four different subjects could be examined for head and neck cancer. For example, it is shown that the staining scores are ranging from 0 to 3 among the breast cancer subjects, from 0 to 3 among the cervical cancer subjects, from 0 to 3 among the colorectal cancer subjects, from 0 to 3 among the ovarian cancer subjects, from 0 to 3 among the testicular cancer subjects and from 0 to 2 among the urothelial cancer subjects. It may thus be concluded that the DACH2 protein is generally differentially expressed in cancer tissue, supporting its role as a treatment predictive marker in various types of cancers.

| **Table 1:** Expression pattern of DACH2 in 20 cancer types | | | | | | | | | | | | |
|---|---|---|---|---|---|---|---|---|---|---|---|---|
| *Cancer type* | **Patient number** | | | | | | | | | | | |
| | **1** | **2** | **3** | **4** | **5** | **6** | **7** | **8** | **9** | **10** | **11** | **12** |
| Breast cancer | 3 | 3 | 3 | 3 | 3 | 3 | 0 | 0 | 0 | - | - | - |
| Cervical cancer | 3 | 1 | 0 | 0 | 0 | 0 | 0 | 0 | 0 | 0 | 0 | 0 |
| Colorectal cancer | 3 | 3 | 3 | 3 | 3 | 3 | 3 | 3 | 3 | 3 | 2 | 0 |
| Endometrial cancer | 3 | 3 | 2 | 2 | 2 | 2 | 2 | 2 | 2 | 0 | 0 | 0 |
| Head & neck cancer | 0 | 0 | 0 | 0 | - | - | - | - | - | - | - | - |
| Liver cancer | 3 | 3 | 0 | 0 | 0 | 0 | 0 | 0 | 0 | 0 | 0 | - |
| Lung cancer | 0 | 0 | 0 | 0 | 0 | 0 | 0 | 0 | 0 | 0 | 0 | 0 |
| Malignant carcinoid | 3 | 3 | 3 | 3 | - | - | - | - | - | - | - | - |
| Malignant glioma | 3 | 1 | 1 | 0 | 0 | 0 | 0 | 0 | 0 | 0 | 0 | 0 |
| Malignant lymphoma | 0 | 0 | 0 | 0 | 0 | 0 | 0 | 0 | 0 | 0 | 0 | 0 |
| Malignant melanoma | 3 | 3 | 3 | 2 | 1 | 0 | 0 | 0 | 0 | 0 | 0 | 0 |
| Ovarian cancer | 3 | 3 | 3 | 3 | 3 | 3 | 3 | 2 | 2 | 1 | 1 | 0 |
| Pancreatic cancer | 1 | 0 | 0 | 0 | 0 | 0 | 0 | 0 | 0 | 0 | 0 | 0 |
| Prostate cancer | 3 | 3 | 3 | 2 | 2 | 2 | 1 | 1 | 0 | 0 | - | - |
| Renal cancer | 3 | 1 | 1 | 0 | 0 | 0 | 0 | 0 | 0 | 0 | - | - |
| Skin cancer | 3 | 3 | 1 | 0 | 0 | 0 | 0 | 0 | 0 | 0 | 0 | 0 |
| Stomach cancer | 3 | 3 | 2 | 1 | 1 | 1 | 1 | 0 | 0 | 0 | 0 | 0 |
| Testis cancer | 3 | 2 | 2 | 2 | 1 | 1 | 0 | 0 | 0 | 0 | - | - |
| Thyroid cancer | 1 | 1 | 0 | 0 | - | - | - | - | - | - | - | - |
| Urothelial cancer | 2 | 1 | 1 | 1 | 1 | 1 | 0 | 0 | 0 | 0 | - | - |

### 2. Ovarian cancer tissue micro array

### a) Material and methods

The cohort is a merge of all incident cases of epithelial ovarian cancers (EOC:s) in the population-based prospective cohort studies Malmö Diet and Cancer Study (n = 101) and Malmö Preventive Medicine Study (n = 108) until Dec 31^{st} 2007. 35 patients participated in both studies, and archival tumor tissue could be retrieved from 154 of the total number of 174 cases. Cases were identified from the Swedish Cancer Registry up until December 31^{st}, 2006, and from The Southern Swedish Regional Tumor Registry for the period of January 1st to December 31st, 2007. All tumors were re-evaluated regarding histological subtype and histological grade by a board certified pathologists. Information regarding clinical stage was obtained from the medical charts, following the standardized FIGO classification of tumor staging. Information on residual tumor after surgery was not available. Standard adjuvant therapy was platinum-based chemotherapy, from the 1990s given in combination with paclitaxel.

Histopathological, clinical and treatment data were obtained from the clinical- and/or pathology records. Information on vital status and cause of death was obtained from the Swedish Cause of Death Registry up until December 31^{st}, 2008. Follow-up started at date of diagnosis and ended at death, emigration or December 31^{st}, 2008, whichever came first. After a median follow-up of 2.65 years (range 0-21), 105 patients (68.2%) were dead and 49 (31.8%) alive. Patient-and tumor characteristics of the cohort have been described in detail. Ethical permissions for the MDCS (Ref. 51/90), and the present study (Ref. 530/2008), were obtained from the Ethics Committee at Lund University.

Areas representative of cancer were marked on full-face haematoxylin and eosin stained sections and TMAs constructed as previously described. In brief, 2-4 1.0 mm cores were taken from each tumor and mounted in a new recipient block using a semi-automated arraying device (TMArrayer; Pathology Devices, Inc, Westminster, MD, USA).

For immunohistochemical analysis of DACH2, 4 µm TMA-sections were automatically pretreated using the PT-link system (DAKO, Copenhagen, Denmark) and then stained in a Techmate 500 (DAKO, Copenhagen, Denmark) with a polyclonal anti-DACH2 antibody (HPA 000258, Atlas Antibodies AB, Stockholm, Sweden) diluted 1:50. The polyclonal anti-DACH2 antibody was generated against SEQ ID NO:1.

DACH2 was primarily expressed in the nucleus, and for assessment of DACH2 expression, both the fraction of positive cells and staining intensity were taken into account. Nuclear fraction (NF) was categorized into four groups, namely 0 (0-1%), 1 (2-25%), 2 (26-75 %) and 3 (> 75%) and nuclear staining intensity (NI) denoted as 0-2, whereby 0 = negative, 1 = intermediate and 2 = moderate-strong intensity. A combined nuclear score (NS) of NF x NI, which had a range of 0 to 6, was then constructed as previously described (Ehlen et al. J Transl Med. 2010 Aug 20;8:78). To check for staining heterogeneity, DACH2 expression was analyzed on a corresponding subset of full-face sections (n=12).

Spearman's Rho test was used for comparison of DACH2 expression and clinicopathological and tumor biological characteristics. Classification regression tree (CRT) analysis was used to decide optimal cutoff for survival analysis. Kaplan-Meier analysis and log rank test were used to illustrate differences in ovarian cancer specific survival (OCSS) and overall survival (OS) according to DACH2 expression. Cox regression proportional hazards models were used for estimation of hazard ratios (HRs) for death from ovarian cancer or overall causes according to DACH2 expression, adjusted for stage and differentiation grade. All calculations were performed using SPSS (SPSS Inc, Chicago, IL).

### b) Results

Following antibody optimization and staining, DACH2 expression could be evaluated in 143/154 (92.9%) of the EOC cases.

There was no significant correlation between DACH2 expression (ns) and established clinicopathological factors, i.e. clinical stage and grade, nor to RBM3 or AR expression.

CRT analysis suggested an optimal cut-off point at NS > 3 to determine the impact of DACH2 expression on OCSS and OS. Kaplan Meier analysis of the subjects in the cohort who are likely to have received platinum-based treatment (n=142), i.e. all subjects but those given no treatment according to the available information, demonstrated a significantly increased OCSS (figures 1A and 2A) and OS (figures 1B and 2B) for subjects showing low levels of DACH2. Further, Kaplan Meier analysis of the subjects in the cohort who are confirmed to have received platinum-based treatment (n=70) demonstrated a clear trend towards increased OCSS (figures 3A) and OS (figures 3B) for tumors expressing low levels of DACH2. The reason for the somewhat higher p values (less significant values) in the latter analysis is probably the relatively low number of subjects involved.

When an alternative cut-off, i.e. NF > 2, was employed for the subjects in the cohort who are likely to have received platinum-based treatment, low levels of DACH2 remained significantly correlated to increased survival

### 3. DACH2 expression in cisplatin sensitive and cisplatin resistant cell lines

### Materials and methods

### a) Cell lines and reagents

The human ovarian cancer cell line A2780 and the cisplatin-resistant variant A2780-Cp70 (received by the inventors as a gift from Prof R Brown, Imperial College, London) were maintained in RPMI-1640 supplemented with glutamine, 10% fetal bovine serum and 1% penicillin/streptomycin in a humidified incubator of 5% CO2 at 37°C. Cisplatin (Sigma-Aldrich, St. Louis, MO, USA) was dissolved in 0.9% NaCl to a stock solution of 1 mg/ml and added to cells to the final concentration (1-100 µM).

### b) Real-time quantitative PCR and Western Blotting

Total RNA isolation (RNeasy, QIAgen, Hilden, Germany), cDNA synthesis (Reverse Transcriptase kit, Applied Biosystems, Warrington, UK) and quantitative real-time PCR (qRT-PCR) analysis with TaqMan Gene Expression Assay targeting DACH2 (Applied Biosystems, Carlsbad, CA) were performed according to the manufacturers instructions. Quantification of expression levels were calculated by using the comparative Ct method, normalization according to house keeping gene 18S from the TaqMan Gene Expression Assay (Applied Biosystems).

For immunoblotting, cells were lysed in ice-cold lysis buffer (150 mM NaCl, 50 mM Tris-HCL pH 7.5, 1 % Triton X-1 00, 50 mM NaF, 1 mM Na3VO4, 1 mM phenylmethylsulfonyl fluoride (PMSF)) and supplemented with protease inhibitor cocktail Complete Mini (Roche, Basel, Switzerland). For Western blotting, 20-50 µg of protein were separated on 15% SDS-PAGE gels and transferred onto nitrocellulose membranes (Hybond ECL, Amersham Pharmacia Biotech, Buckinghamshire, UK). The membranes were probed with primary antibodies followed by horseradish peroxidase (HRP)-conjugated secondary antibodies (Amersham Life Science, Alesbury, U.K.) and visualized using the Enhanced ChemiLuminescence detection system (ECL) and ECL films (Amersham Pharmacia Biotech). DACH2 was detected by the polyclonal DACH2 antibody (HPA 000258, Atlas Antibodies AB, Stockholm, Sweden) diluted 1:250 in blocking solution (5% BSA, 1 x PBS, 0.1 % Tween20). Membranes were stripped and re-probed with an anti-β-actin antibody (Santa Cruz, Biotechnology, Santa Cruz, CA, USA) at a dilution of 1:1000, to provide a loading control.

### c) Cell pellet arrays

Cell lines were fixed in 4% formalin and processed in gradient alcohols. Cell pellets were cleared in xylene and washed multiple times in molten paraffin. Once processed, cell lines were arrayed in duplicate 1.0 mm cores using a manual tissue arrayer (Beecher Inc, WI) and IHC was performed on 4 µm sections using the anti-DACH2 antibody described above (HPA 000258, Atlas Antibodies AB, Stockholm, Sweden).

### d) siRNA knockdown of DACH2 gene expression

Transfection with siRNA against DACH2 (Applied Biosystems, Carlsbad, Ca) or control siRNA (Applied Biosystems) was performed with Lipofectamine 2000 (Invitrogen, Carlsbad, CA) with a final concentration of 50 nM siRNA. All siRNA experiments were performed using 3 independent RNA oligonucleotides (Pre-designed siRNA: ID, s229510, s229511, s229512) targeting DACH2.

### Results

### a) Validation of the DACH2 antibody and immunohistochemical expression of DACH2

The specificity of the DACH2 antibody was confirmed by siRNA-mediated knockdown of DACH2 in A2780 and A2780/Cp70 cells. IHC performed on formalin fixed, paraffin embedded siRNA transfected A2780 and A2780/CP70 cells revealed a marked decrease in immunoreactivity in the DACH2 knockdown cells compared to controls as visualized by IHC (not shown).

### b) DACH2 levels are higher in cisplatin-resistant than cisplatin-sensitive ovarian cancer cells

DACH2 protein expression, assessed by both Western blotting (Figure 5) and IHC, was higher in the cisplatin-resistant derivative A2780-Cp70 cells compared to the parental A2780 cells. Real-time quantitative PCR (qRT-PCR) confirmed a similar difference whereby there was an almost 3-fold higher level of DACH2 mRNA in the A2780-Cp70 compared to the A2780 cell line (Figure 6). Cisplatin resistance in the A2780-Cp70 cells relative to the A2780 cells has been confirmed previously (Ehlen et al. J Transl Med. 2010 Aug 20;8:78).

### Establishment of a treatment prediction

### 4. A non-limiting example

Following the establishment of a cancer diagnosis (e.g. an ovarian, a testicular or a colorectal cancer) in a patient, a tumor tissue sample from the patient is obtained. The tumor tissue sample may be obtained from a specimen from a surgical removal of the tumor or from a tumor biopsy. Further, for the provision of a "negative reference", a sample is taken from archival material comprising tissue showing low, or essentially lacking, DACH2 protein expression. Such archival tissue may for example be tissue having a pre-established low DACH2 protein expression level from the same cancer type as the one of the tested subject. Further, for the provision of a "positive reference", a sample is taken from archival material comprising tissue showing high DACH2 protein expression, also from the same cancer type as the one of the tested subject.

The sample material is fixated in buffered formalin and histo-processed in order to obtain thin sections (4 µm) of the sample material.

Immunohistochemistry is performed in line with what is described in section 2 above. One or more sample section(s) from each sample is/are mounted on glass slides that are incubated for 45 min in 60 °C, de-paraffinized (if the sample in question was paraffinized) in xylene (2 x 15 min) and hydrated in graded alcohols. For antigen retrieval, slides are immersed in TRS (Target Retrieval Solution, pH 6.0, DakoCytomation) and boiled for 4 min at 125°C in a Decloaking chamber® (Biocare Medical). Slides are placed in the Autostainer® (DakoCytomation) and endogenous peroxidase is initially blocked with H2O2 (DakoCytomation). The reason for mounting multiple sample sections is to increase the accuracy of the results.

A primary DACH2 protein specific antibody (e.g. HPA000258, Atlas Antibodies AB, Stockholm, Sweden) is added to the slides, which are then incubated for 30 min in room temperature, followed by 30 min of incubation in room temperature with a labeled secondary antibody. To detect the secondary antibody, diaminobenzidine (DakoCytomation) is used as chromogen, contrasted with a Harris hematoxylin (Sigma-Aldrich) counterstaining. Between all steps, slides are rinsed in wash buffer (DakoCytomation). The slides are then mounted with Pertex® (Histolab) mounting media.

Optionally, two control cell-lines may be used as a tool to validate the staining procedure; e.g. one slide with cells expressing DACH2 protein (positive cell line) and one slide having cells with indistinct weak or no DACH2 protein expression (negative cell line). The control-line slides may be simultaneously stained in the same procedure as the other slides, i.e. incubated with the same primary and secondary antibodies.

For example, the tumor tissue slides from the subject, the staining reference slides, and optionally, the slides with control cell-lines, may be scanned in a light microscope using a ScanScope T2 automated slide scanning system (Aperio Technologies) at x20 magnification. However, this scanning step is not necessary, but may make the procedure easier if, for example, the preparation and staining of the slides and the evaluation of the stained slides (see below) are performed at different locations or by different persons.

If control cell-lines are used, these are inspected to validate the staining procedure. If the cell-lines display staining results outside acceptable criteria, e.g. staining artifacts recognized by the skilled artisan, the staining of the tissue samples is considered invalid and the whole staining procedure is repeated with new slides. If the positive and negative cell-lines display strong staining intensity and indistinct weak or no staining intensity, respectively, the staining is considered as valid.

The stained sample slide(s) from the tumor tissue is/are evaluated manually by visual inspection in accordance to standards used in clinical histo-pathological diagnostics, and the immunoreactivity of the cancer slide(s) is/are graded in accordance with what is described in section 2 above.

That is, the nuclear intensity and/or the nuclear fraction is/are determined to obtain one or more sample values. The person performing the evaluation and determination is aided by visual inspection of the stained positive and negative reference slides.

The sample value(s) from the tumor tissue sample from the patient is/are then compared to a reference value. If more than one sample slide are evaluated and thereby more than one sample value are obtained, the sample value that is compared to the reference value may be a mean or median value of the obtained sample values.

The reference value may for example be a nuclear score (NS) of 3 (see section 2 above).

If the sample value(s) is/are lower than or equal to the reference value, the physician responsible for the treatment of the patient concludes that the patient's responsiveness to platinum-based treatment is relatively high and optionally, that the patient should be given a platinum-based treatment.

If however the sample value(s) is/are higher than the reference value, the physician concludes that the patient's responsiveness to platinum-based treatment is relatively low and, optionally, that the patient should be given a platinum-based treatment of a higher intensity (such as a higher dose) if the patient suffers from testicular or ovarian cancer and to refrain from platinum-based treatment if the subject suffers from any other type of cancer.

## Claims

1. Method for determining whether a mammalian subject having a cancer belongs to a first or a second group, wherein subjects of the first group are more responsive to a platinum-based treatment than subjects of the second group, comprising the steps of:
a) evaluating an amount of Dachshund homolog 2 (DACH2) present in at least part of a sample earlier obtained from said subject, and determining a sample value corresponding to said amount;
b) comparing the sample value obtained in step a) with a reference value; and,
if said sample value is lower than or equal to said reference value,
c1) concluding that said subject belongs to said first group; and
if said sample value is higher than said reference value,
c2) concluding that said subject belongs to said second group.

2. Method for determining the level of intensity of a platinum-based treatment of a mammalian subject having a cancer, comprising the steps of:
a) evaluating an amount of DACH2 present in at least part of a sample earlier obtained from said subject, and determining a sample value corresponding to said amount;
b) comparing the sample value obtained in step a) with a reference value; and,
if said sample value is lower than or equal to said reference value,
c1) concluding that said subject should be given a platinum-based treatment of said first intensity; and
if said sample value is higher than or equal to said reference value,
c2) concluding that said subject should be given a platinum-based treatment of said second intensity,
wherein said second intensity is higher than said first intensity.

3. Method according to claim 1 or 2, wherein said cancer is selected from testicular, ovarian, lung, bladder, colorectal, cervical, breast and head and neck cancer.

4. Method according to claim 3, wherein said cancer is selected from testicular, ovarian, bladder, colorectal, cervical and breast cancer.

5. Method according to any one of the preceding claims, wherein said platinum-based treatment is selected from carboplatin, paraplatin, oxaliplatin, satraplatin, picoplatin and cisplatin treatment.

6. Method according to any one of claims 1-5, wherein the evaluation of step a) is limited to the nuclei of tumor cells of said sample.

7. Method according to any one of the preceding claims, wherein step a) comprises:
al) applying to said sample a quantifiable affinity ligand capable of selective interaction with a DACH2 protein to be evaluated, said application being performed under conditions that enable binding of the affinity ligand to DACH2 protein present in the sample; and
all) quantifying the affinity ligand bound to said sample to evaluate said amount.

8. Kit comprising:
a) a quantifiable affinity ligand capable of selective interaction with a DACH2 protein; and
b) reagents necessary for quantifying an amount of said quantifiable affinity ligand.

9. Kit according to claim 8, in which said quantifiable affinity ligand is capable of selective interaction with a DACH2 fragment consisting of SEQ ID NO:1.

10. Use *ex vivo* of a DACH2 protein as a treatment predictive marker for platinum-based treatment of a mammalian subject having a cancer.

11. Use *ex vivo* of a DACH2 protein or an antigenically active fragment thereof for the selection or purification of a treatment predictive agent for platinum-based treatment of a mammalian subject having a cancer.

12. Use *ex vivo* of a DACH2 protein or an antigenically active fragment thereof for the production of a treatment predictive agent for platinum-based treatment of a mammalian subject having a cancer.

13. Use *ex vivo* of an affinity ligand capable of selective interaction with a DACH2 protein as a treatment predictive agent for platinum-based treatment of a mammalian subject having a cancer.

14. Use *ex vivo* of an affinity ligand capable of selective interaction with a DACH2 protein for indicating whether a mammalian subject having a cancer should be given a platinum-based treatment.

15. Platinum-based therapeutic agent, such as cisplatin or oxaliplatin, for use in treatment of a mammalian subject having a cancer, such as a testicular, an ovarian or a colorectal cancer, wherein said subject is diagnosed as DACH2 low.


SEQUENCE LISTING

<110>  Atlas Antibodies AB

<120>  NEW PREDICTION OF RESPONSE TO PLATINUM-BASED THERAPY

<130>  11584EP

<160>  5

<170>  PatentIn version 3.5

<210>  1
<211>  119
<212>  PRT
<213>  Homo sapiens

<400>  1

Ile Thr Ala Ala Ala Met Ala Glu Ala Met Lys Leu Gln Lys Met Lys
1               5                   10                  15

Leu Met Ala Met Asn Thr Leu Gln Gly Asn Gly Ser Gln Asn Gly Thr
            20                  25                  30

Glu Ser Glu Pro Asp Asp Leu Asn Ser Asn Thr Gly Gly Ser Glu Ser
        35                  40                  45

Ser Trp Asp Lys Asp Lys Met Gln Ser Pro Phe Ala Ala Pro Gly Pro
    50                  55                  60

Gln His Gly Ile Ala His Ala Ala Leu Ala Gly Gln Pro Gly Ile Gly
65                  70                  75                  80

Gly Ala Pro Thr Leu Asn Pro Leu Gln Gln Asn His Leu Leu Thr Asn
                85                  90                  95

Arg Leu Asp Leu Pro Phe Met Met Met Pro His Pro Leu Leu Pro Val
            100                 105                 110

Ser Leu Pro Pro Ala Ser Val
        115

<210>  2
<211>  432
<212>  PRT
<213>  Homo sapiens

<400>  2

Met Thr Arg Lys Gln Ala Val Asn Ser Ser Arg Pro Gly Arg Pro Pro
1               5                   10                  15

Lys Arg Ser Leu Gly Val Leu Gln Glu Asn Ala Arg Leu Leu Thr His
            20                  25                  30

Ala Val Pro Gly Leu Leu Ser Pro Gly Leu Ile Thr Pro Thr Gly Ile
        35                  40                  45

Thr Ala Ala Ala Met Ala Glu Ala Met Lys Leu Gln Lys Met Lys Leu
    50                  55                  60

Met Ala Met Asn Thr Leu Gln Gly Asn Gly Ser Gln Asn Gly Thr Glu
65                  70                  75                  80

Ser Glu Pro Asp Asp Leu Asn Ser Asn Thr Gly Gly Ser Glu Ser Ser
                85                  90                  95

Trp Asp Lys Asp Lys Met Gln Ser Pro Phe Ala Ala Pro Gly Pro Gln
            100                 105                 110

His Gly Ile Ala His Ala Ala Leu Ala Gly Gln Pro Gly Ile Gly Gly
        115                 120                 125

Ala Pro Thr Leu Asn Pro Leu Gln Gln Asn His Leu Leu Thr Asn Arg
    130                 135                 140

Leu Asp Leu Pro Phe Met Met Met Pro His Pro Leu Leu Pro Val Ser
145                 150                 155                 160

Leu Pro Pro Ala Ser Val Ala Met Ala Met Asn Gln Met Asn His Leu
                165                 170                 175

Asn Thr Ile Ala Asn Met Ala Ala Ala Ala Gln Ile His Ser Pro Leu
            180                 185                 190

Ser Arg Ala Gly Thr Ser Val Ile Lys Glu Arg Ile Pro Glu Ser Pro
        195                 200                 205

Ser Pro Ala Pro Ser Leu Glu Glu Asn His Arg Pro Gly Ser Gln Thr
    210                 215                 220

Ser Ser His Thr Ser Ser Ser Val Ser Ser Ser Pro Ser Gln Met Asp
225                 230                 235                 240

His His Leu Glu Arg Met Glu Glu Val Pro Val Gln Ile Pro Ile Met
                245                 250                 255

Lys Ser Pro Leu Asp Lys Ile Gln Leu Thr Pro Gly Gln Ala Leu Pro
            260                 265                 270

Ala Gly Phe Pro Gly Pro Phe Ile Phe Ala Asp Ser Leu Ser Ser Val
        275                 280                 285

Glu Thr Leu Leu Thr Asn Ile Gln Gly Leu Leu Lys Val Ala Leu Asp
    290                 295                 300

Asn Ala Arg Ile Gln Glu Lys Gln Ile Gln Gln Glu Lys Lys Glu Leu
305                 310                 315                 320

Arg Leu Glu Leu Tyr Arg Glu Arg Glu Ile Arg Glu Asn Leu Glu Arg
                325                 330                 335

Gln Leu Ala Val Glu Leu Gln Ser Arg Thr Thr Met Gln Lys Arg Leu
            340                 345                 350

Lys Lys Glu Lys Lys Thr Lys Arg Lys Leu Gln Glu Ala Leu Glu Phe
        355                 360                 365

Glu Ser Lys Arg Arg Glu Gln Val Glu Gln Ala Leu Lys Gln Ala Thr
    370                 375                 380

Thr Ser Asp Ser Gly Leu Arg Met Leu Lys Asp Thr Gly Ile Pro Asp
385                 390                 395                 400

Ile Glu Ile Glu Asn Asn Gly Thr Pro His Asp Ser Ala Ala Met Gln
                405                 410                 415

Gly Gly Asn Tyr Tyr Cys Leu Glu Met Ala Gln Gln Leu Tyr Ser Ala
            420                 425                 430

<210>  3
<211>  599
<212>  PRT
<213>  Homo sapiens

<400>  3

Met Ala Val Ser Ala Ser Pro Val Ile Ser Ala Thr Ser Ser Gly Ala
1               5                   10                  15

Gly Val Pro Gly Gly Leu Phe Arg Ala Glu Pro Leu Tyr Ser Thr Pro
            20                  25                  30

Arg Glu Pro Pro Arg Leu Thr Pro Asn Met Ile Asn Ser Phe Val Val
        35                  40                  45

Asn Asn His Ser Asn Ser Ala Gly Gly Gly Gly Arg Gly Asn Thr Asn
    50                  55                  60

Thr Asn Glu Cys Arg Met Val Asp Met His Gly Met Lys Val Ala Ser
65                  70                  75                  80

Phe Leu Met Asp Gly Gln Glu Leu Ile Cys Leu Pro Gln Val Phe Asp
                85                  90                  95

Leu Phe Leu Lys His Leu Val Gly Gly Leu His Thr Val Tyr Thr Lys
            100                 105                 110

Leu Lys Arg Leu Asp Ile Ser Pro Val Val Cys Thr Val Glu Gln Val
        115                 120                 125

Arg Ile Leu Arg Gly Leu Gly Ala Ile Gln Pro Gly Val Asn Arg Cys
    130                 135                 140

Lys Leu Ile Thr Arg Lys Asp Phe Glu Thr Leu Phe Thr Asp Cys Thr
145                 150                 155                 160

Asn Ala Arg Arg Lys Arg Gln Met Thr Arg Lys Gln Ala Val Asn Ser
                165                 170                 175

Ser Arg Pro Gly Arg Pro Pro Lys Arg Ser Leu Gly Val Leu Gln Glu
            180                 185                 190

Asn Ala Arg Leu Leu Thr His Ala Val Pro Gly Leu Leu Ser Pro Gly
        195                 200                 205

Leu Ile Thr Pro Thr Gly Ile Thr Ala Ala Ala Met Ala Glu Ala Met
    210                 215                 220

Lys Leu Gln Lys Met Lys Leu Met Ala Met Asn Thr Leu Gln Gly Asn
225                 230                 235                 240

Gly Ser Gln Asn Gly Thr Glu Ser Glu Pro Asp Asp Leu Asn Ser Asn
                245                 250                 255

Thr Gly Gly Ser Glu Ser Ser Trp Asp Lys Asp Lys Met Gln Ser Pro
            260                 265                 270

Phe Ala Ala Pro Gly Pro Gln His Gly Ile Ala His Ala Ala Leu Ala
        275                 280                 285

Gly Gln Pro Gly Ile Gly Gly Ala Pro Thr Leu Asn Pro Leu Gln Gln
    290                 295                 300

Asn His Leu Leu Thr Asn Arg Leu Asp Leu Pro Phe Met Met Met Pro
305                 310                 315                 320

His Pro Leu Leu Pro Val Ser Leu Pro Pro Ala Ser Val Ala Met Ala
                325                 330                 335

Met Asn Gln Met Asn His Leu Asn Thr Ile Ala Asn Met Ala Ala Ala
            340                 345                 350

Ala Gln Ile His Ser Pro Leu Ser Arg Ala Gly Thr Ser Val Ile Lys
        355                 360                 365

Glu Arg Ile Pro Glu Ser Pro Ser Pro Ala Pro Ser Leu Glu Glu Asn
    370                 375                 380

His Arg Pro Gly Ser Gln Thr Ser Ser His Thr Ser Ser Ser Val Ser
385                 390                 395                 400

Ser Ser Pro Ser Gln Met Asp His His Leu Glu Arg Met Glu Glu Val
                405                 410                 415

Pro Val Gln Ile Pro Ile Met Lys Ser Pro Leu Asp Lys Ile Gln Leu
            420                 425                 430

Thr Pro Gly Gln Ala Leu Pro Ala Gly Phe Pro Gly Pro Phe Ile Phe
        435                 440                 445

Ala Asp Ser Leu Ser Ser Val Glu Thr Leu Leu Thr Asn Ile Gln Gly
    450                 455                 460

Leu Leu Lys Val Ala Leu Asp Asn Ala Arg Ile Gln Glu Lys Gln Ile
465                 470                 475                 480

Gln Gln Glu Lys Lys Glu Leu Arg Leu Glu Leu Tyr Arg Glu Arg Glu
                485                 490                 495

Ile Arg Glu Asn Leu Glu Arg Gln Leu Ala Val Glu Leu Gln Ser Arg
            500                 505                 510

Thr Thr Met Gln Lys Arg Leu Lys Lys Glu Lys Lys Thr Lys Arg Lys
        515                 520                 525

Leu Gln Glu Ala Leu Glu Phe Glu Ser Lys Arg Arg Glu Gln Val Glu
    530                 535                 540

Gln Ala Leu Lys Gln Ala Thr Thr Ser Asp Ser Gly Leu Arg Met Leu
545                 550                 555                 560

Lys Asp Thr Gly Ile Pro Asp Ile Glu Ile Glu Asn Asn Gly Thr Pro
                565                 570                 575

His Asp Ser Ala Ala Met Gln Gly Gly Asn Tyr Tyr Cys Leu Glu Met
            580                 585                 590

Ala Gln Gln Leu Tyr Ser Ala
        595

<210>  4
<211>  571
<212>  PRT
<213>  Homo sapiens

<400>  4

Met Ala Val Ser Ala Ser Pro Val Ile Ser Ala Thr Ser Ser Gly Ala
1               5                   10                  15

Gly Val Pro Gly Gly Leu Phe Arg Ala Glu Pro Leu Tyr Ser Thr Pro
            20                  25                  30

Arg Glu Pro Pro Arg Leu Thr Pro Asn Met Ile Asn Ser Phe Val Val
        35                  40                  45

Asn Asn His Ser Asn Ser Ala Gly Gly Gly Gly Arg Gly Asn Thr Asn
    50                  55                  60

Thr Asn Glu Cys Arg Met Val Asp Met His Gly Met Lys Val Ala Ser
65                  70                  75                  80

Phe Leu Met Asp Gly Gln Glu Leu Ile Cys Leu Pro Gln Val Phe Asp
                85                  90                  95

Leu Phe Leu Lys His Leu Val Gly Gly Leu His Thr Val Tyr Thr Lys
            100                 105                 110

Leu Lys Arg Leu Asp Ile Ser Pro Val Val Cys Thr Val Glu Gln Val
        115                 120                 125

Arg Ile Leu Arg Gly Leu Gly Ala Ile Gln Pro Gly Val Asn Arg Cys
    130                 135                 140

Lys Leu Ile Thr Arg Lys Asp Phe Glu Thr Leu Phe Thr Asp Cys Thr
145                 150                 155                 160

Asn Ala Ser Ser Arg Pro Gly Arg Pro Pro Lys Arg Ser Leu Gly Val
                165                 170                 175

Leu Gln Glu Asn Ala Arg Leu Leu Thr His Ala Val Pro Gly Leu Leu
            180                 185                 190

Ser Pro Gly Leu Ile Thr Pro Thr Gly Ile Thr Ala Ala Ala Met Ala
        195                 200                 205

Glu Ala Met Lys Leu Gln Lys Met Lys Leu Met Ala Met Asn Thr Leu
    210                 215                 220

Gln Gly Asn Gly Ser Gln Asn Gly Thr Glu Ser Glu Pro Asp Asp Leu
225                 230                 235                 240

Asn Ser Asn Thr Gly Gly Ser Glu Ser Ser Trp Asp Lys Asp Lys Met
                245                 250                 255

Gln Ser Pro Phe Ala Ala Pro Gly Pro Gln His Gly Ile Ala His Ala
            260                 265                 270

Ala Leu Ala Gly Gln Pro Gly Ile Gly Gly Ala Pro Thr Leu Asn Pro
        275                 280                 285

Leu Gln Gln Asn His Leu Leu Thr Asn Arg Leu Asp Leu Pro Phe Met
    290                 295                 300

Met Met Pro His Pro Leu Leu Pro Val Ser Leu Pro Pro Ala Ser Val
305                 310                 315                 320

Ala Met Ala Met Asn Gln Met Asn His Leu Asn Thr Ile Ala Asn Met
                325                 330                 335

Ala Ala Ala Ala Gln Ile His Ser Pro Leu Ser Arg Ala Gly Thr Ser
            340                 345                 350

Val Ile Lys Glu Arg Ile Pro Glu Ser Pro Ser Pro Ala Pro Ser Leu
        355                 360                 365

Glu Glu Asn His Arg Pro Gly Ser Gln Thr Ser Ser His Thr Ser Ser
    370                 375                 380

Ser Val Ser Ser Ser Pro Ser Gln Met Asp His His Leu Glu Arg Met
385                 390                 395                 400

Glu Glu Val Pro Val Gln Ile Pro Ile Met Lys Ser Pro Leu Asp Lys
                405                 410                 415

Ile Gln Leu Thr Pro Gly Gln Ala Leu Pro Ala Gly Phe Pro Gly Pro
            420                 425                 430

Phe Ile Phe Ala Asp Ser Leu Ser Ser Val Glu Thr Leu Leu Thr Asn
        435                 440                 445

Ile Gln Gly Leu Leu Lys Val Ala Leu Asp Asn Ala Arg Ile Gln Glu
    450                 455                 460

Lys Gln Ile Gln Gln Glu Lys Lys Glu Leu Arg Leu Glu Leu Tyr Arg
465                 470                 475                 480

Glu Arg Glu Ile Arg Glu Asn Leu Glu Arg Gln Leu Ala Val Glu Leu
                485                 490                 495

Gln Ser Arg Thr Thr Met Gln Lys Arg Leu Lys Lys Glu Lys Lys Thr
            500                 505                 510

Lys Arg Lys Leu Gln Glu Ala Leu Glu Phe Glu Ser Lys Arg Arg Glu
        515                 520                 525

Gln Val Glu Gln Ala Leu Lys Gln Ala Thr Thr Ser Asp Ser Gly Leu
    530                 535                 540

Arg Met Leu Lys Asp Thr Gly Ile Pro Asp Ile Glu Ile Glu Asn Asn
545                 550                 555                 560

Gly Thr Pro His Asp Ser Ala Ala Met Gln Ala
                565                 570

<210>  5
<211>  380
<212>  PRT
<213>  Homo sapiens

<400>  5

Met Ala Glu Ala Met Lys Leu Gln Lys Met Lys Leu Met Ala Met Asn
1               5                   10                  15

Thr Leu Gln Gly Asn Gly Ser Gln Asn Gly Thr Glu Ser Glu Pro Asp
            20                  25                  30

Asp Leu Asn Ser Asn Thr Gly Gly Ser Glu Ser Ser Trp Asp Lys Asp
        35                  40                  45

Lys Met Gln Ser Pro Phe Ala Ala Pro Gly Pro Gln His Gly Ile Ala
    50                  55                  60

His Ala Ala Leu Ala Gly Gln Pro Gly Ile Gly Gly Ala Pro Thr Leu
65                  70                  75                  80

Asn Pro Leu Gln Gln Asn His Leu Leu Thr Asn Arg Leu Asp Leu Pro
                85                  90                  95

Phe Met Met Met Pro His Pro Leu Leu Pro Val Ser Leu Pro Pro Ala
            100                 105                 110

Ser Val Ala Met Ala Met Asn Gln Met Asn His Leu Asn Thr Ile Ala
        115                 120                 125

Asn Met Ala Ala Ala Ala Gln Ile His Ser Pro Leu Ser Arg Ala Gly
    130                 135                 140

Thr Ser Val Ile Lys Glu Arg Ile Pro Glu Ser Pro Ser Pro Ala Pro

145                 150                 155                 160

Ser Leu Glu Glu Asn His Arg Pro Gly Ser Gln Thr Ser Ser His Thr
                165                 170                 175

Ser Ser Ser Val Ser Ser Ser Pro Ser Gln Met Asp His His Leu Glu
            180                 185                 190

Arg Met Glu Glu Val Pro Val Gln Ile Pro Ile Met Lys Ser Pro Leu
        195                 200                 205

Asp Lys Ile Gln Leu Thr Pro Gly Gln Ala Leu Pro Ala Gly Phe Pro
    210                 215                 220

Gly Pro Phe Ile Phe Ala Asp Ser Leu Ser Ser Val Glu Thr Leu Leu
225                 230                 235                 240

Thr Asn Ile Gln Gly Leu Leu Lys Val Ala Leu Asp Asn Ala Arg Ile
                245                 250                 255

Gln Glu Lys Gln Ile Gln Gln Glu Lys Lys Glu Leu Arg Leu Glu Leu
            260                 265                 270

Tyr Arg Glu Arg Glu Ile Arg Glu Asn Leu Glu Arg Gln Leu Ala Val
        275                 280                 285

Glu Leu Gln Ser Arg Thr Thr Met Gln Lys Arg Leu Lys Lys Glu Lys
    290                 295                 300

Lys Thr Lys Arg Lys Leu Gln Glu Ala Leu Glu Phe Glu Ser Lys Arg
305                 310                 315                 320

Arg Glu Gln Val Glu Gln Ala Leu Lys Gln Ala Thr Thr Ser Asp Ser
                325                 330                 335

Gly Leu Arg Met Leu Lys Asp Thr Gly Ile Pro Asp Ile Glu Ile Glu
            340                 345                 350

Asn Asn Gly Thr Pro His Asp Ser Ala Ala Met Gln Gly Gly Asn Tyr
        355                 360                 365

Tyr Cys Leu Glu Met Ala Gln Gln Leu Tyr Ser Ala
    370                 375                 380